# EUROPEAN PATENT APPLICATION

(11) **EP 4 129 973 A1**
(43) Date of publication of application: **08.02.2023**
(21) Application number: 22192109.1
(22) Date of filing: 11.03.2019
(51) Int. Cl.: C07C 303/34, C01B 21/086, C07C 307/02, C07C 311/48, C07D 295/26, C01B 17/98, C01B 21/096, C07C 303/40

(54) **LITHIUM SULFAMATE DERIVATIVES**

(30) Priority: 27.03.2018 JP 2018060824
(62) Divisional of application: 19777318.7
(71) Applicant: Daikin Industries, Ltd., Osaka-shi, Osaka 530-0001 (JP)
(72) Inventor: Suzuki, Yuuki, Osaka-shi, Osaka, 530-8323 (JP); Yamauchi, Akiyoshi, Osaka-shi, Osaka, 530-8323 (JP); Hidaka, Tomoya, Osaka-shi, Osaka, 530-8323 (JP); Hayashi, Kotaro, Osaka-shi, Osaka, 530-8323 (JP); Kuwajima, Yoshiko, Osaka-shi, Osaka, 530-8323 (JP); Yamamoto, Yoshihiro, Osaka-shi, Osaka, 530-8323 (JP); Kishikawa, Yosuke, Osaka-shi, Osaka, 530-8323 (JP)
(74) Representative: Hoffmann Eitle

(57) **Abstract**

The invention provides a compound of formula (3a): 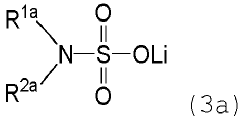 wherein R^{1a} and R^{2a} each independently are C₁₋₇-fluoroalkyl, C₂₋₇-alkenyl or C₂₋₇-alkynyl, each optionally fluorinated; -SO₂X wherein X is H, F or optionally fluorinated alkyl; or cyanomethyl; and R^{1a} and R^{2a} each independently may contain at least one of O, S and N atoms in the structure.

## Description

### TECHNICAL FIELD

The disclosure relates to methods for producing a lithium sulfamate, and novel lithium sulfamates.

### BACKGROUND ART

Patent Literature 1 discloses that morpholine-4-sodium sulfamate was obtained by the procedure including: adding triethylamine to morpholine chloride; stirring the mixture at room temperature for 15 minutes; adding chloroform thereto; cooling the mixture to -5°C; dropwise adding chlorosulfonic acid to the mixture while maintaining the temperature at 0°C or lower; and treating the mixture with a sodium hydroxide aqueous solution.

Non-Patent Literature 1 discloses that diethylsulfamic acid was synthesized by reacting diethylamine with chlorosulfonic acid.

### CITATION LIST

### - Patent Literature

Patent Literature 1: WO 2006/077414

### - Non-Patent Literature

Non-Patent Literature 1: Chemical Communications 43, 2016, pp. 7032-7035

### SUMMARY OF INVENTION

### - Technical Problem

The disclosure aims to provide a method for producing a lithium sulfamate, and a novel lithium sulfamate.

### - Solution to problem

The disclosure relates to a method for producing a lithium sulfamate, including
(1) reacting a compound (1) represented by the following formula (1): 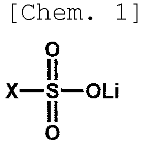 wherein X is fluorine, chlorine, bromine, or iodine, with a compound (2) represented by the following formula (2) : 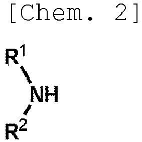 wherein R¹ and R² are each individually a substituent that is:
   - H;
      a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1;
      a C1-C7 alkyl group;
      a C2-C7 alkenyl group;
      a C2-C7 alkynyl group;
      a C6-C15 aryl group;
   - SO₂X¹ wherein X¹ is -H, -F, or an alkyl group in which at least one hydrogen atom is optionally replaced with a fluorine atom;
   - SO₃X² wherein X² is -H, -F, or an alkyl group in which at least one hydrogen atom is optionally replaced with a fluorine atom; or
      a C2-C7 hydrocarbon group that forms a cyclic structure by bonding of R¹ and R², the cyclic structure optionally containing a multiple bond,
      the substituent optionally containing at least one bi- to hexavalent heteroatom in the structure and being a substituent in which at least one hydrogen atom is optionally replaced with a fluorine atom or a C0-C7 functional group, to obtain a compound (3) represented by the following formula (3): 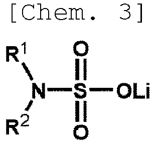 wherein R¹ and R² are defined as above (hereafter, also referred to as "the production method of a first aspect of the disclosure").

The production method of the first aspect of the disclosure preferably further includes (2) reacting a compound (4) represented by the following formula (4): 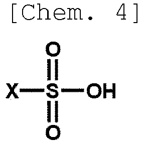 wherein X is fluorine, chlorine, bromine, or iodine, with a lithium source to obtain the compound (1) represented by the following formula (1): 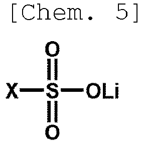 wherein X is fluorine, chlorine, bromine, or iodine.

The lithium source is preferably lithium fluoride, lithium chloride, lithium bromide, lithium iodide, lithium hydride, lithium hydroxide, or metallic lithium, more preferably lithium fluoride, lithium chloride, lithium bromide, or lithium iodide.

The disclosure also relates to a method for producing a lithium sulfamate, including
(3) reacting a compound (4) represented by the following formula (4): 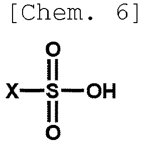 wherein X is fluorine, chlorine, bromine, or iodine, with a compound (5) represented by the following formula (5) : 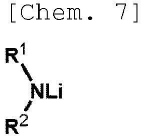 wherein R¹ and R² are each individually a substituent that is:
- H;
   a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1;
   a C1-C7 alkyl group;
   a C2-C7 alkenyl group;
   a C2-C7 alkynyl group;
   a C6-C15 aryl group;
- SO₂X¹ wherein X¹ is -H, -F, or an alkyl group in which at least one hydrogen atom is optionally replaced with a fluorine atom;
- SO₃X² wherein X² is -H, -F, or an alkyl group in which at least one hydrogen atom is optionally replaced with a fluorine atom; or
   a C2-C7 hydrocarbon group that forms a cyclic structure by bonding of R¹ and R², the cyclic structure optionally containing a multiple bond,
   the substituent optionally containing at least one bi- to hexavalent heteroatom in the structure and being a substituent in which at least one hydrogen atom is optionally replaced with a fluorine atom or a C0-C7 functional group, to obtain a compound (3) represented by the following formula (3): 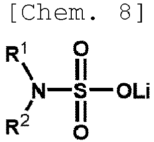 wherein R¹ and R² are defined as above. (hereafter, also referred to as "the production method of a second aspect of the disclosure").

At least one selected from R¹ and R² in the compound (5) represented by the formula (5) is preferably a trifluoromethyl group, a 2,2,2-trifluoroethyl group, a pentafluoroethyl group, a 2,2,3,3,3-pentafluoropropyl group, a heptafluoropropyl group, a fluorosulfonyl group, a trifluoromethanesulfonyl group, a 2,2,2-trifluoroethanesulfonyl group, a pentafluoroethanesulfonyl group, a 2,2,3,3,3-pentafluoropropanesulfonyl group, a heptafluoropropanesulfonyl group, or a cyanomethyl group.

The disclosure relates to a compound represented by the following formula (3a): 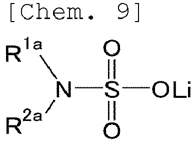 wherein R^{1a} and R^{2a} are each individually a substituent that is:
- H;
   a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom; n is an integer of 0 or greater; and p is 0 or 1;
   a C1-C7 alkyl group;
   a C2-C7 alkenyl group;
   a C2-C7 alkynyl group;
- SO₂X¹ wherein X¹ is -H, -F, or an alkyl group in which at least one hydrogen atom is optionally replaced with a fluorine atom;
- SO₃X² wherein X² is -H, -F, or an alkyl group in which at least one hydrogen atom is optionally replaced with a fluorine atom; or
   a C2-C7 hydrocarbon group that forms a cyclic structure by bonding of R^{1a} and R^{2a}, the cyclic structure optionally containing a multiple bond,
   the substituent optionally containing at least one bi- to hexavalent heteroatom in the structure and being a substituent in which at least one hydrogen atom is optionally replaced with a fluorine atom or a C0-C7 functional group, and
   when R^{1a} and R^{2a} are both alkyl groups, at least one alkyl group contains at least one bi- to hexavalent heteroatom in the structure or is an alkyl group in which at least one hydrogen atom is optionally replaced with a fluorine atom or a C0-C7 functional group.

In the formula (3a), preferably, R^{1a} and R^{2a} are each individually a substituent that is:
a C1-C7 alkyl group in which at least one hydrogen atom is replaced with a fluorine atom;
a C2-C7 alkenyl group in which at least one hydrogen atom is optionally replaced with a fluorine atom;
a C2-C7 alkynyl group in which at least one hydrogen atom is optionally replaced with a fluorine atom;
   - SO₂X wherein X is -H, -F, or an alkyl group in which at least one hydrogen atom is optionally replaced with a fluorine atom;
a cyanomethyl group; or
a C2-C7 hydrocarbon group that forms a cyclic structure by bonding of R^{1a} and R^{2a}, the cyclic structure optionally containing a multiple bond and at least one hydrogen atom therein being optionally replaced with a fluorine atom,
the substituent optionally containing at least one selected from oxygen, sulfur, and nitrogen atoms in the structure.

### - Advantageous Effects of Invention

The production method of the disclosure can provide a lithium sulfamate. The disclosure provides a novel lithium sulfamate.

### DESCRIPTION OF EMBODIMENTS

The method for producing a lithium sulfamate and a novel lithium sulfamate of the disclosure are specifically described in the following.

The production method of the first aspect of the disclosure includes (1) reacting a compound (1) represented by the following formula (1): 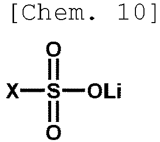 wherein X is fluorine, chlorine, bromine, or iodine, with a compound (2) represented by the following formula (2) : 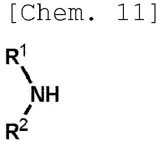 wherein R¹ and R² are each individually a substituent that is:
- H;
   a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1;
   a C1-C7 alkyl group;
   a C2-C7 alkenyl group;
   a C2-C7 alkynyl group;
   a C6-C15 aryl group;
- SO₂X¹ wherein X¹ is -H, -F, or an alkyl group in which at least one hydrogen atom is optionally replaced with a fluorine atom;
- SO₃X² wherein X² is -H, -F, or an alkyl group in which at least one hydrogen atom is optionally replaced with a fluorine atom; or
   a C2-C7 hydrocarbon group that forms a cyclic structure by bonding of R¹ and R², the cyclic structure optionally containing a multiple bond,
   the substituent optionally containing at least one bi- to hexavalent heteroatom in the structure and being a substituent in which at least one hydrogen atom is optionally replaced with a fluorine atom or a C0-C7 functional group, to obtain a compound (3) represented by the following formula (3): 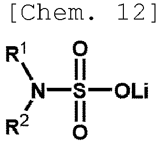 wherein R¹ and R² are defined as above.

The production method of the first aspect of the disclosure can provide a novel lithium sulfamate owing to the above configuration. The use of the compound (1) that is a lithium salt reduces the heat of reaction with the compound (2) that is a base, enabling simple and efficient production of a lithium sulfamate. The production method is also beneficial in that hardly separable by-products, such as lithium sulfate, are not likely to be generated.

The substituent indicates -H, the group represented by the formula: -Oₚ-(SiR³₂O)ₙ-SiR⁴₃, the alkyl group, the alkenyl group, the alkynyl group, the aryl group, the - SO₂X¹, the -SO₃X², or the hydrocarbon group.

The substituent optionally contains at least one bi-to hexavalent heteroatom or is a substituent in which at least one hydrogen atom is replaced with a fluorine atom or a C0-C7 functional group.

The functional group optionally contained in the substituent is preferably a phenyl group, an anisyl group, a benzyl group, a cyano group, a trialkylsilyl group (alkyl group preferably contains 1 to 4 carbon atoms), -SO₂X³ (X³ is -H, -F, or an alkyl group in which at least one hydrogen atom is optionally replaced with a fluorine atom), a C1-C7 alkyl group in which at least one hydrogen atom is optionally replaced with a fluorine atom, a C1-C7 saturated heterocyclic group, or a C1-C7 alkoxy group. The alkyl group in X³ contains, for example, 1 to 10 carbon atoms.

In R¹ and R², the alkyl group may be a linear, branched, or cyclic group, and preferably contains 1 to 10, more preferably 1 to 7 carbon atoms. The alkyl group may be a fluoroalkyl group in which a hydrogen atom bonded to a carbon atom is replaced with a fluorine atom or a group in which a hydrogen atom bonded to a carbon atom is replaced with the above functional group.

In R¹ and R², the alkenyl group may be a linear, branched, or cyclic group, and preferably contains 2 to 10, more preferably 2 to 7 carbon atoms. The alkenyl group may be a fluoroalkenyl group in which a hydrogen atom bonded to a carbon atom is replaced with a fluorine atom or a group in which a hydrogen atom bonded to a carbon atom is replaced with the above functional group.

In R¹ and R², the alkynyl group may be a linear, branched, or cyclic group, and preferably contains 2 to 10, more preferably 2 to 7 carbon atoms. The alkynyl group may be a fluoroalkynyl group in which a hydrogen atom bonded to a carbon atom is replaced with a fluorine atom or a group in which a hydrogen atom bonded to a carbon atom is replaced with the above functional group.

In the R¹ and R², the aryl group preferably contains 6 or 7 carbon atoms. The aryl group may be a fluoroaryl group in which a hydrogen atom bonded to a carbon atom is replaced with a fluorine atom or a group in which a hydrogen atom bonded to a carbon atom is replaced with the above functional group.

R¹ and R² each may be a group represented by the formula: -Oₚ-(SiR³₂O)ₙ-SiR⁴₃ (R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1).

In R³ and R⁴, the alkyl group in which at least one hydrogen atom is optionally replaced with a fluorine atom contains preferably 1 to 10, more preferably 1 to 7 carbon atoms.

The alkenyl and alkynyl groups in which at least one hydrogen atom is optionally replaced with a fluorine atom each preferably contain 2 to 10, more preferably 2 to 7 carbon atoms.

The aryl group in which at least one hydrogen atom is optionally replaced with a fluorine atom preferably contains 6 to 8, more preferably 6 or 7 carbon atoms.

In the formula, n is an integer of 0 or greater, preferably 2000 or smaller, more preferably 0 to 100, still more preferably 0 to 10.

R¹ and R² each may be a group represented by -SO₂X¹ (X¹ is -H, -F, or an alkyl group in which at least one hydrogen atom is optionally replaced with a fluorine atom). The alkyl group in the -SO₂X¹ group preferably contains 1 to 10, more preferably 1 to 7 carbon atoms.

R¹ and R² each may be a group represented by -SO₃X² (X² is -H, -F, or an alkyl group in which at least one hydrogen atom is optionally replaced with a fluorine atom). The alkyl group in the -SO₃X² group preferably contains 1 to 10, more preferably 1 to 7 carbon atoms.

Specific examples of R¹ and R² include, but are not limited to, acyclic alkyl groups such as methyl, ethyl, n-propyl, i-propyl, n-butyl, i-butyl, sec-butyl, tert-butyl, pentyl, i-pentyl, neopentyl, sec-pentyl, 3-pentyl, tert-pentyl, and hexyl groups; cyclic alkyl groups such as cyclopentyl, cyclohexyl, norbornanyl, and 1-adamantyl groups; alkenyl groups such as vinyl, 1-propenyl, 2-propenyl (allyl), 2-butenyl, and 1,3-butadienyl groups; alkynyl groups such as ethynyl, 1-propynyl, 2-propynyl, and 2-butynyl groups; halogenated alkyl groups such as trifluoromethyl, 2,2,2-trifluoroethyl, 1,1,2,2-tetrafluoroethyl, pentafluoroethyl, 2,2,3,3,3-pentafluoropropyl, 1,1,2,3,3,3-hexafluoropropyl, and heptafluoropropyl groups; halogenated alkenyl groups such as 1-fluorovinyl, and 2-fluoroallyl groups; alkyl groups having a functional group such as a cyanomethyl group; alkyl groups having a saturated heterocyclic group such as a 3-pyrrolizinopropyl group; aryl groups such as a phenyl group optionally having an alkyl substituent or an alkoxy substituent; aralkyl groups such as phenylmethyl and phenylethyl groups; trialkylsilyl groups such as a trimethylsilyl group; trialkylsiloxy groups such as a trimethylsiloxy group; and sulfonyl groups such as fluorosulfonyl, trifluoromethanesulfonyl, and pentafluoroethanesulfonyl groups.

In the case where R¹ and R² are the hydrocarbon groups forming a cyclic structure by bonding thereof, for example, R¹ and R² may form, together with a nitrogen atom (N) in the formula (2), a cyclic amino group, such as a pyrrolidino or piperidino group or form a heteroatom-containing cyclic amino group such as a 4-morpholino, succinimidyl, or maleimidyl group containing a heteroatom. In these groups, at least one hydrogen atom bonded to a carbon atom is optionally replaced with a fluorine atom, or a hydrogen atom bonded to a carbon atom is optionally replaced with the above functional group. The cyclic structure optionally contains a double or triple bond.

The substituent optionally contains a bi- to hexavalent heteroatom. Examples of the heteroatom include an oxygen atom (O), a sulfur atom (S), a nitrogen atom (N), a silicon atom (Si), a phosphorus atom (P), and a boron atom (B). More preferred is an oxygen atom, a sulfur atom, or a nitrogen atom.

Specific examples of the compound (2) include the following primary amines: 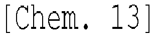 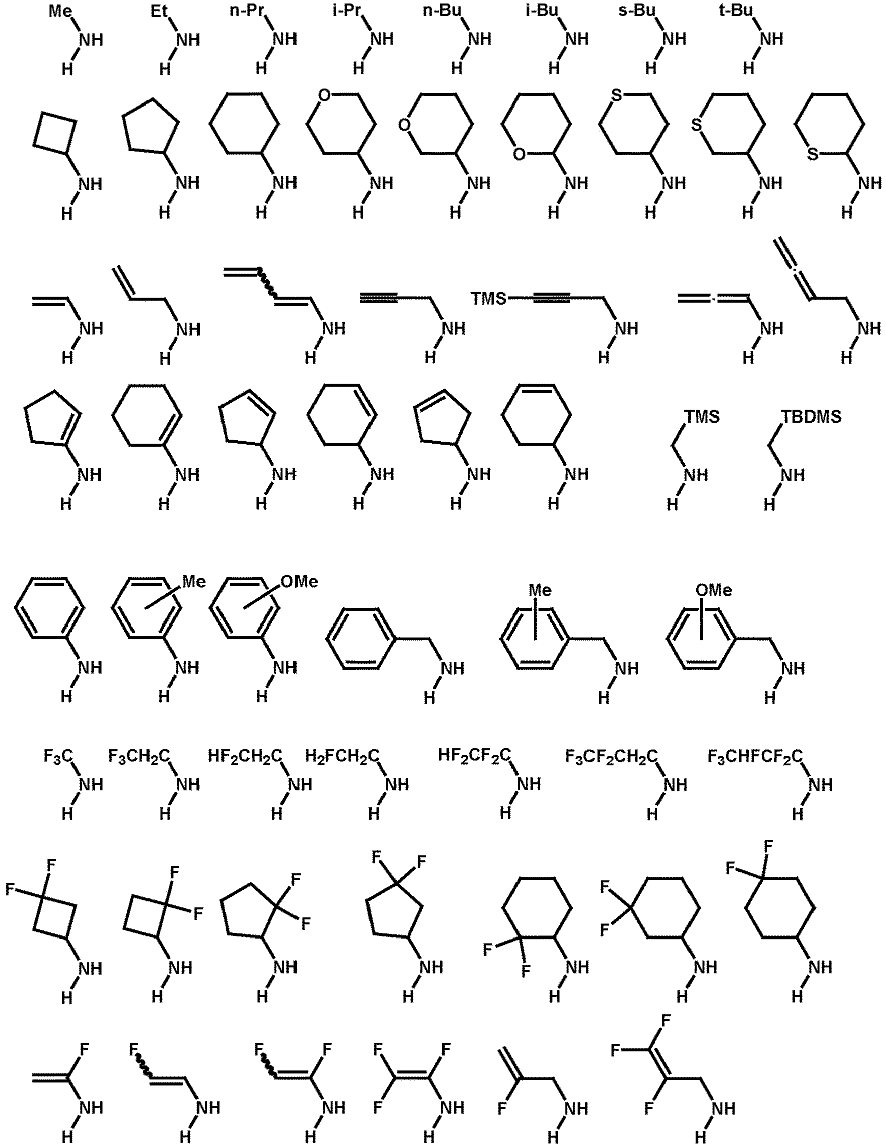 and the following secondary amines:

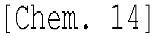

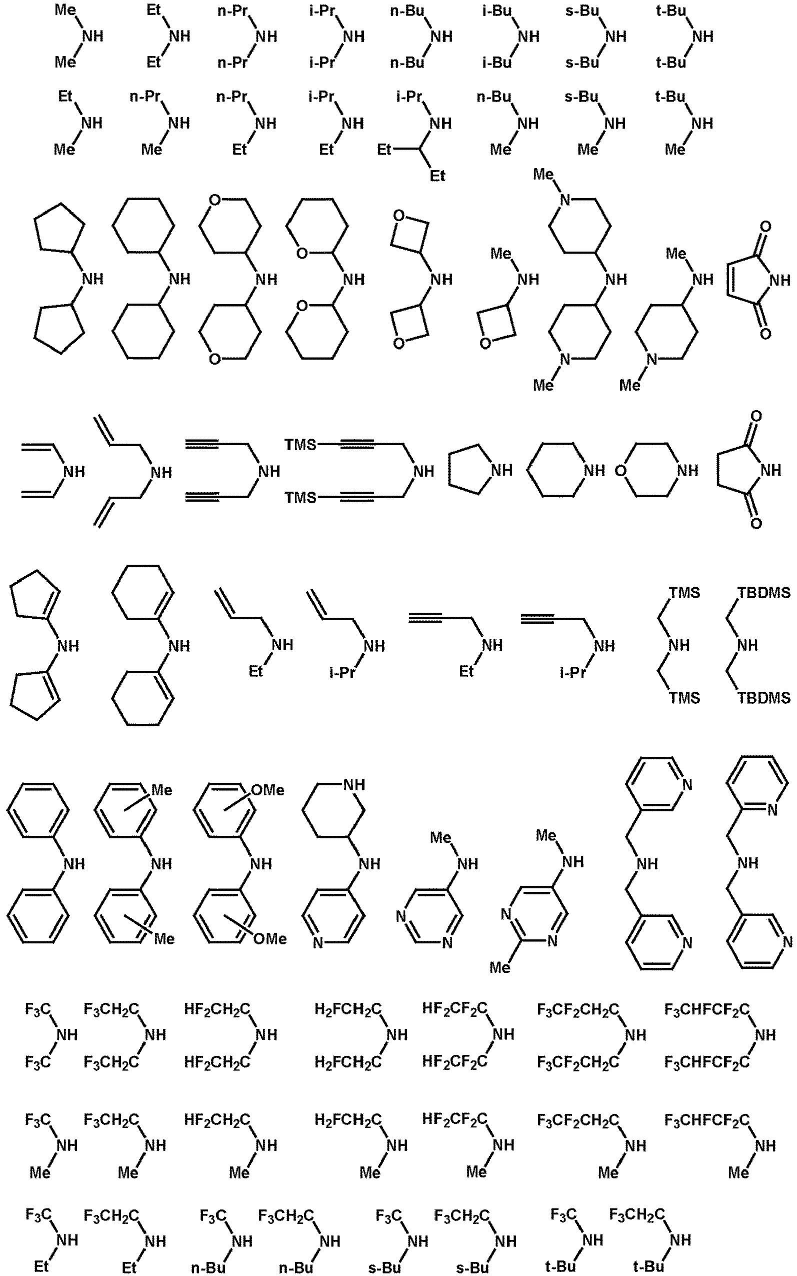

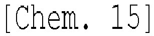

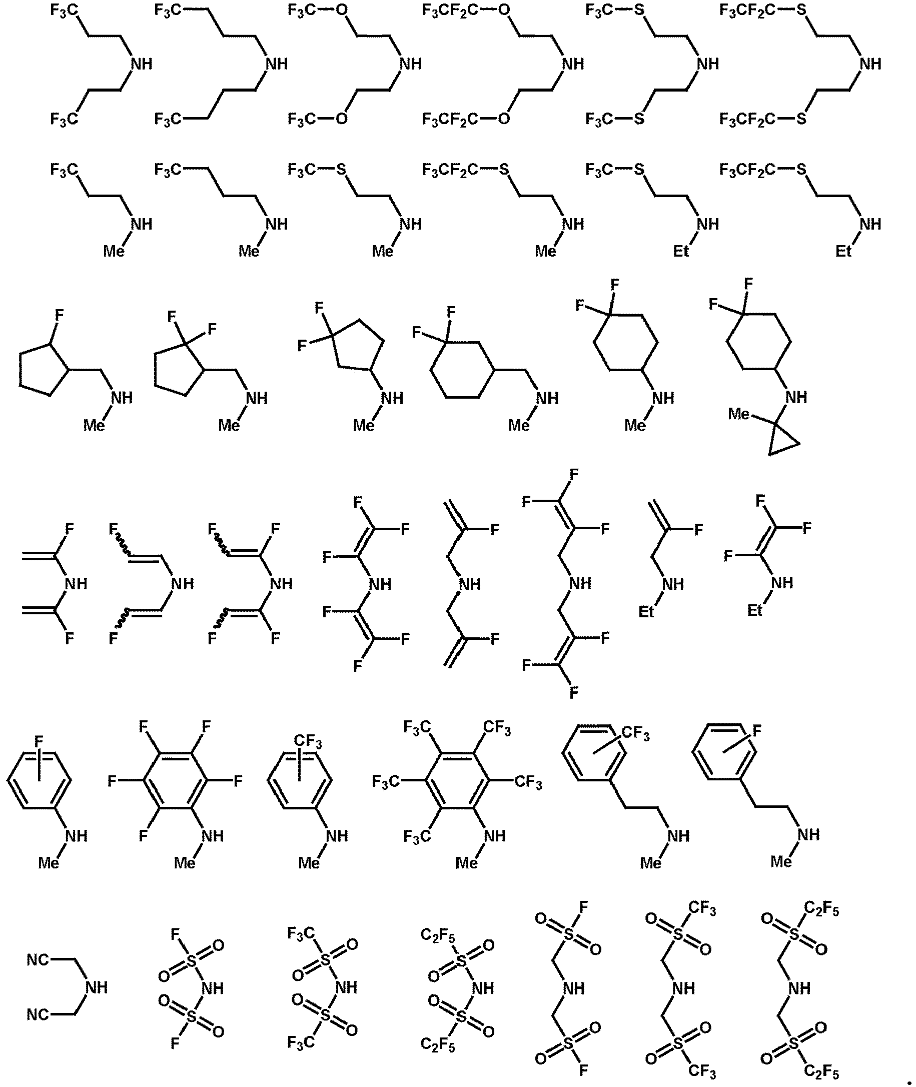

Herein, Me represents a methyl group, Et represents an ethyl group, n-Pr represents a normal propyl group, i-Pr represents an isopropyl group, n-Bu represents a normal butyl group, i-Bu represents an iso-butyl group, s-Bu represents a sec-butyl group, t-Bu represents a tert-butyl group, TMS represents a trimethylsilyl group, and TBDMS represents a tert-butyldimethylsilyl group. In the case of the following benzene ring, R may be bonded to any carbon atom constituting the benzene ring, that is, the benzene ring may have R at any of o-, m-, and p-positions. Examples of compounds herein are not limited to the described specific examples and include geometric isomers (if present) of the compounds.

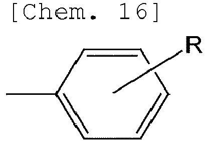

Specific examples of the compound (3) include the following compounds.

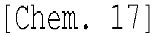

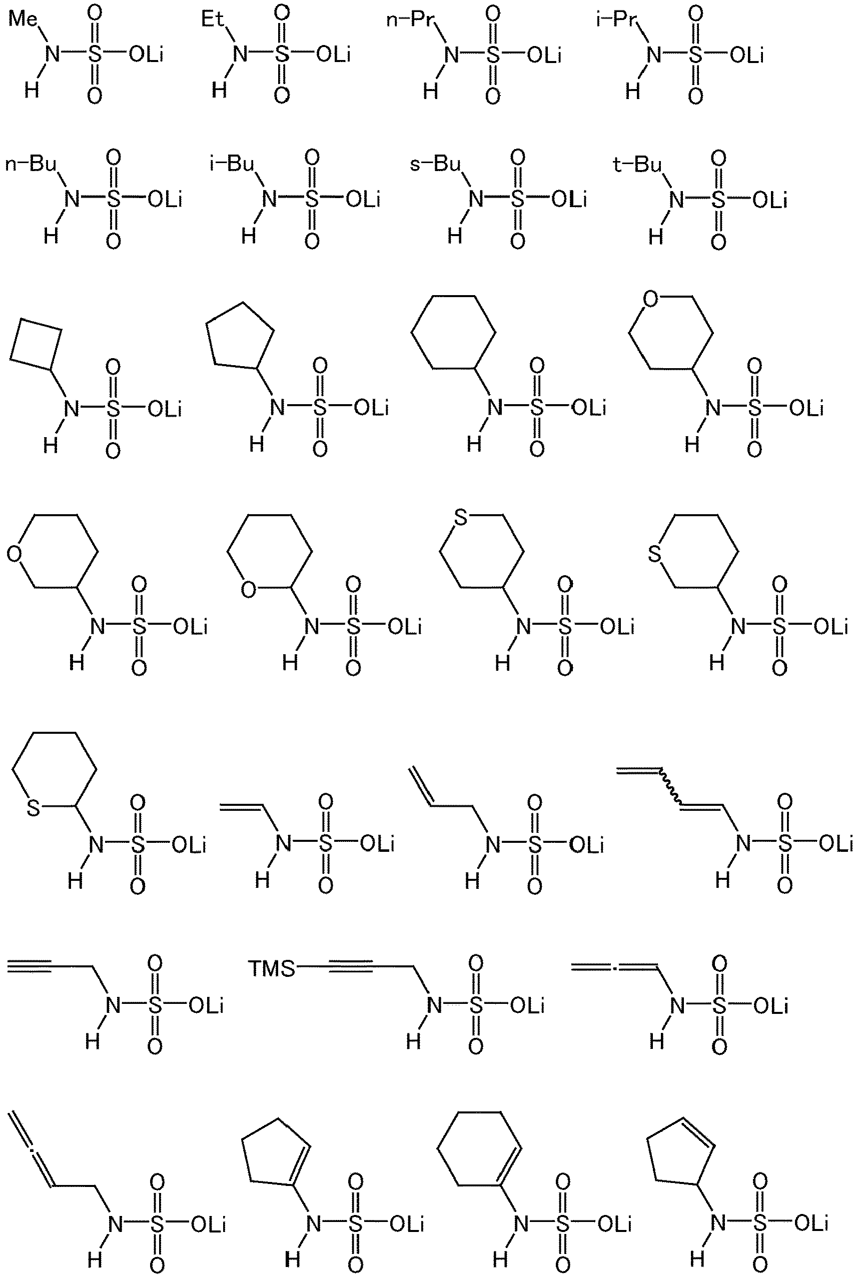

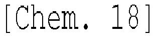

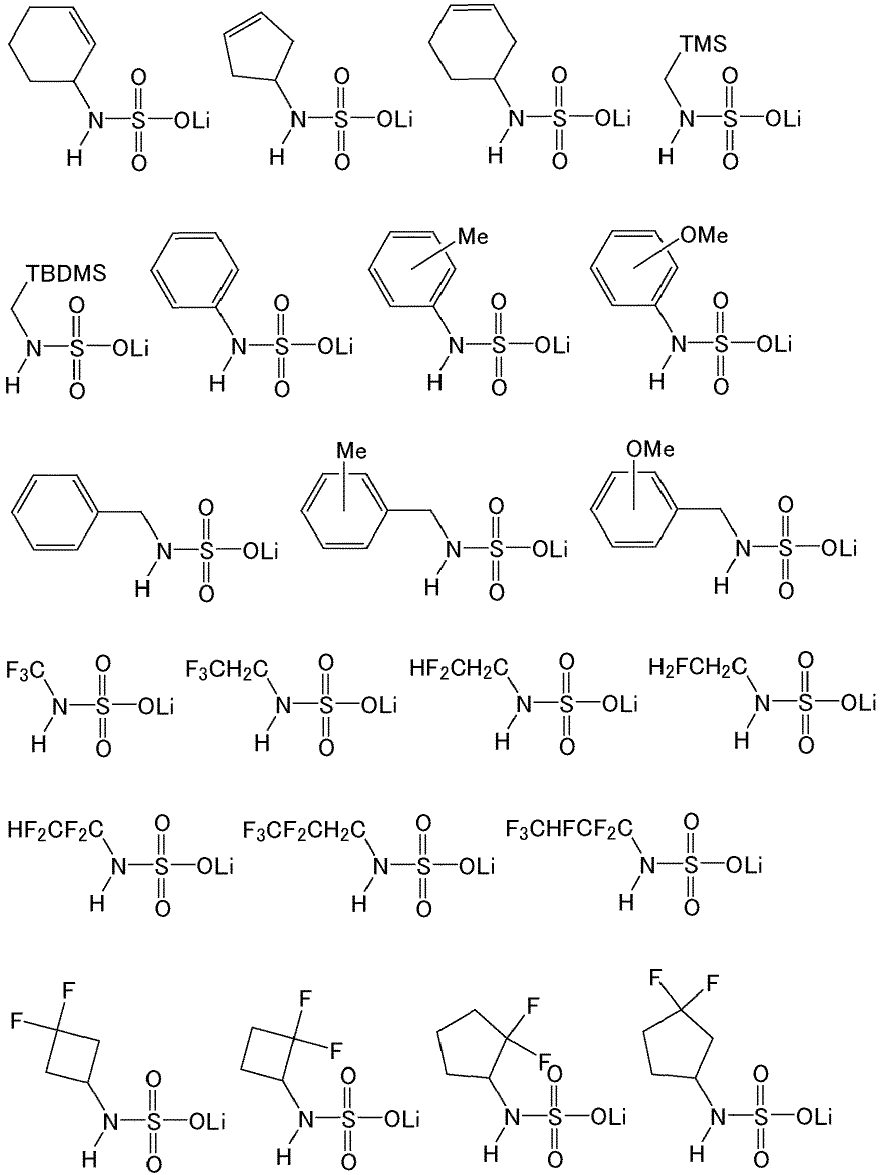

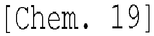

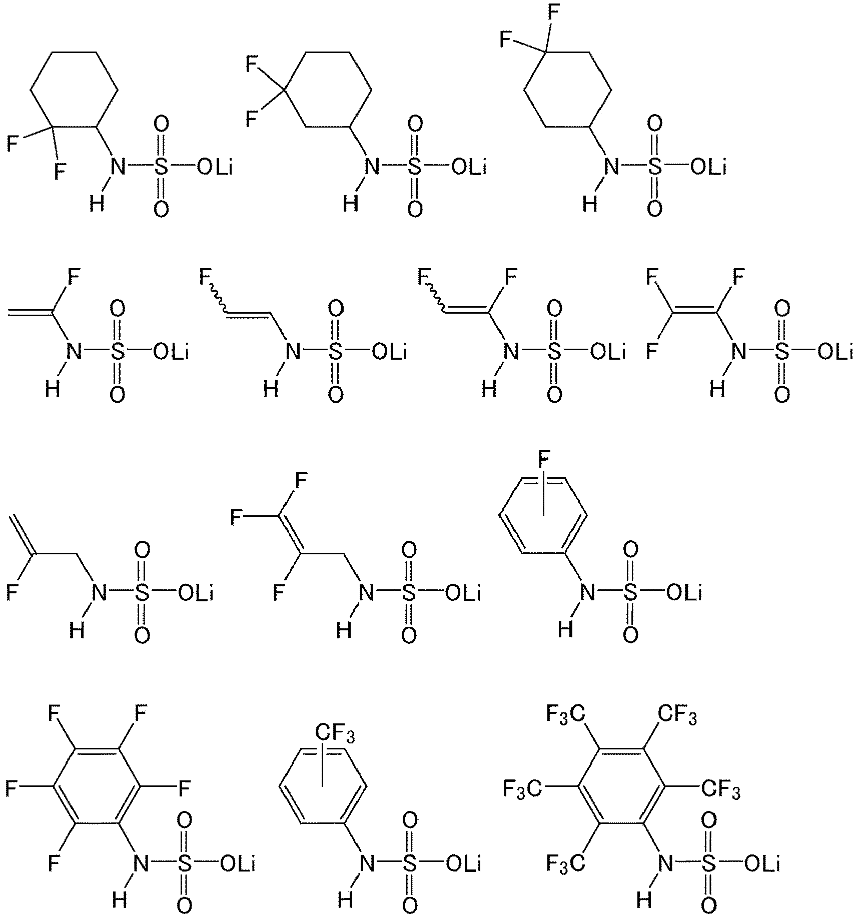

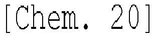

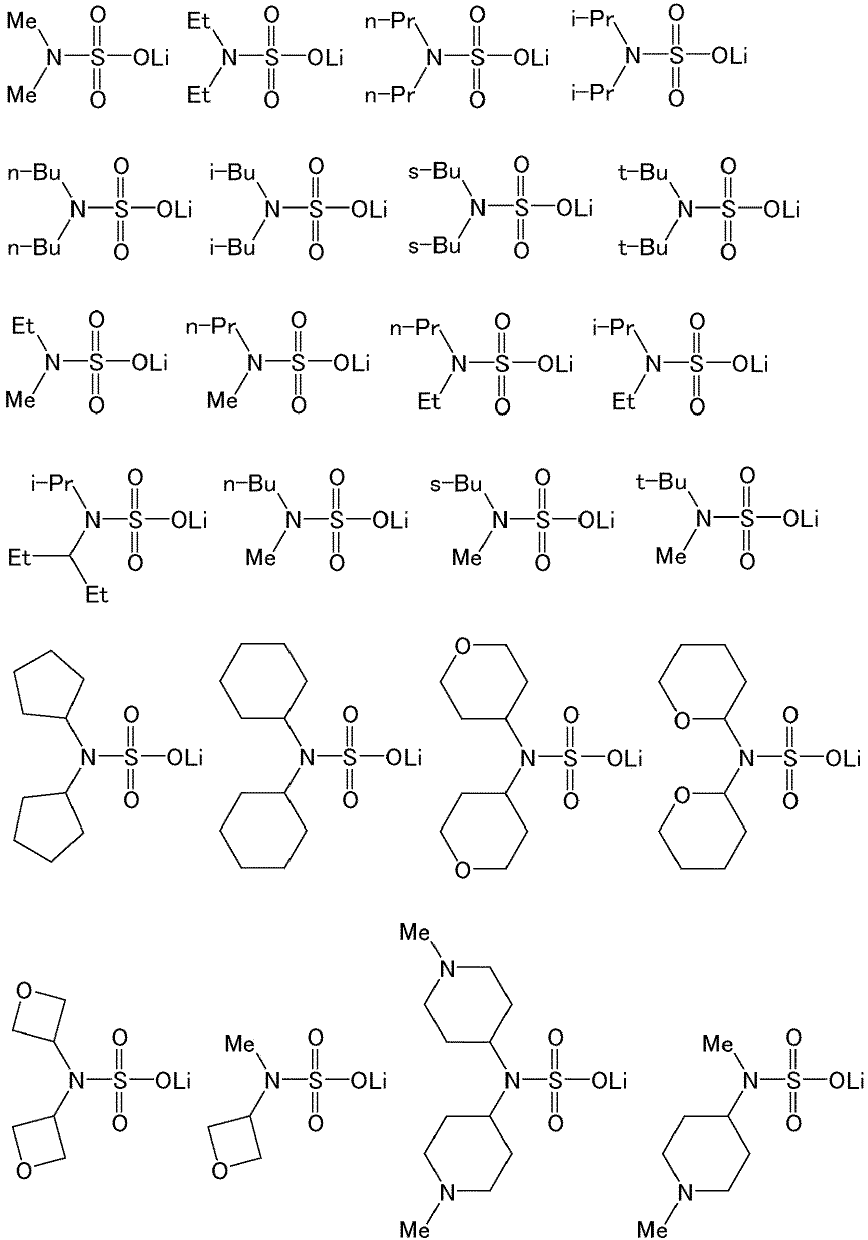

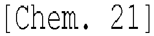

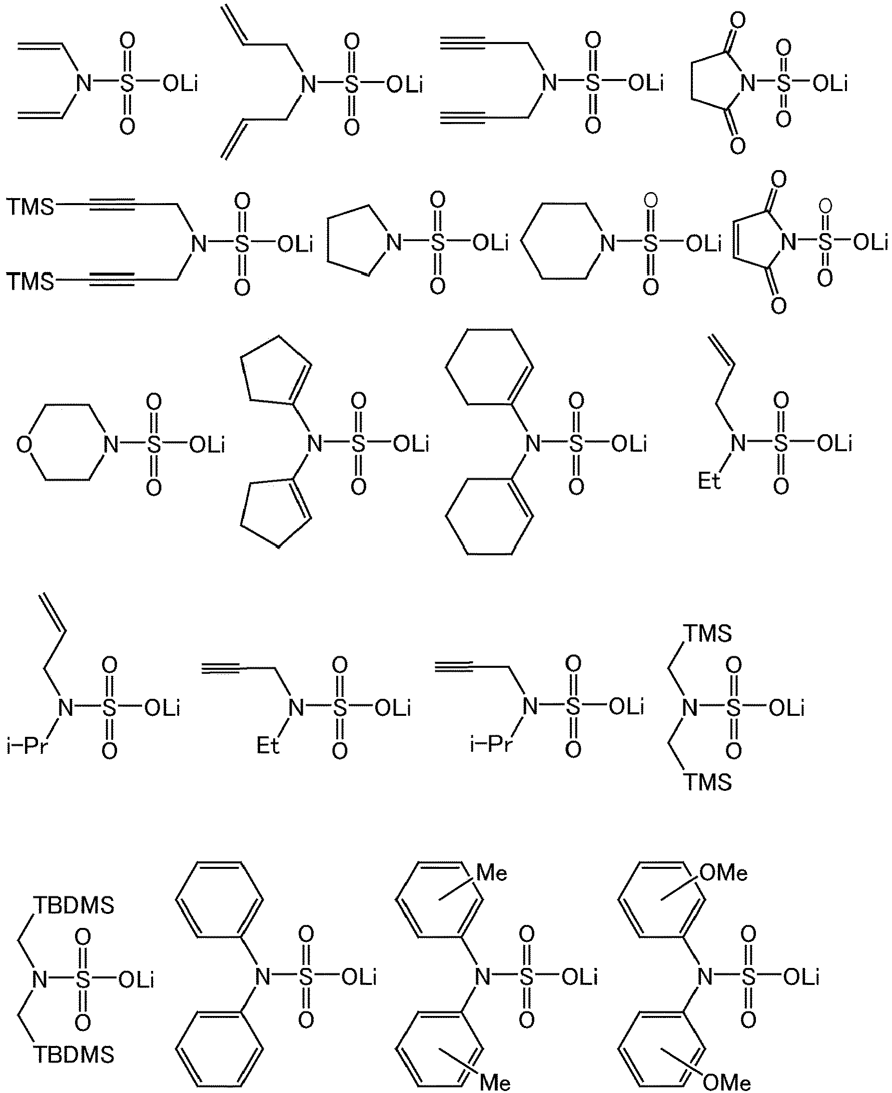

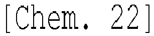

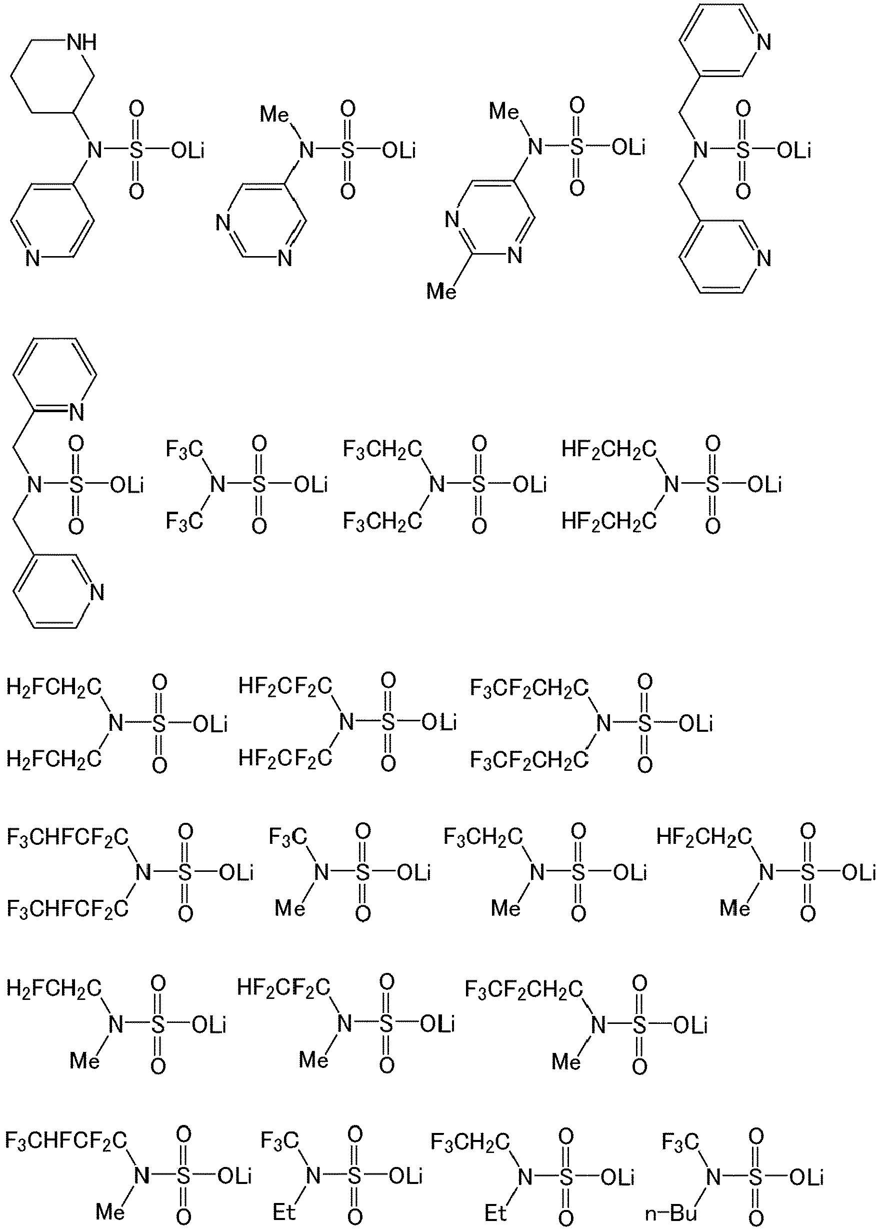

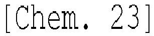

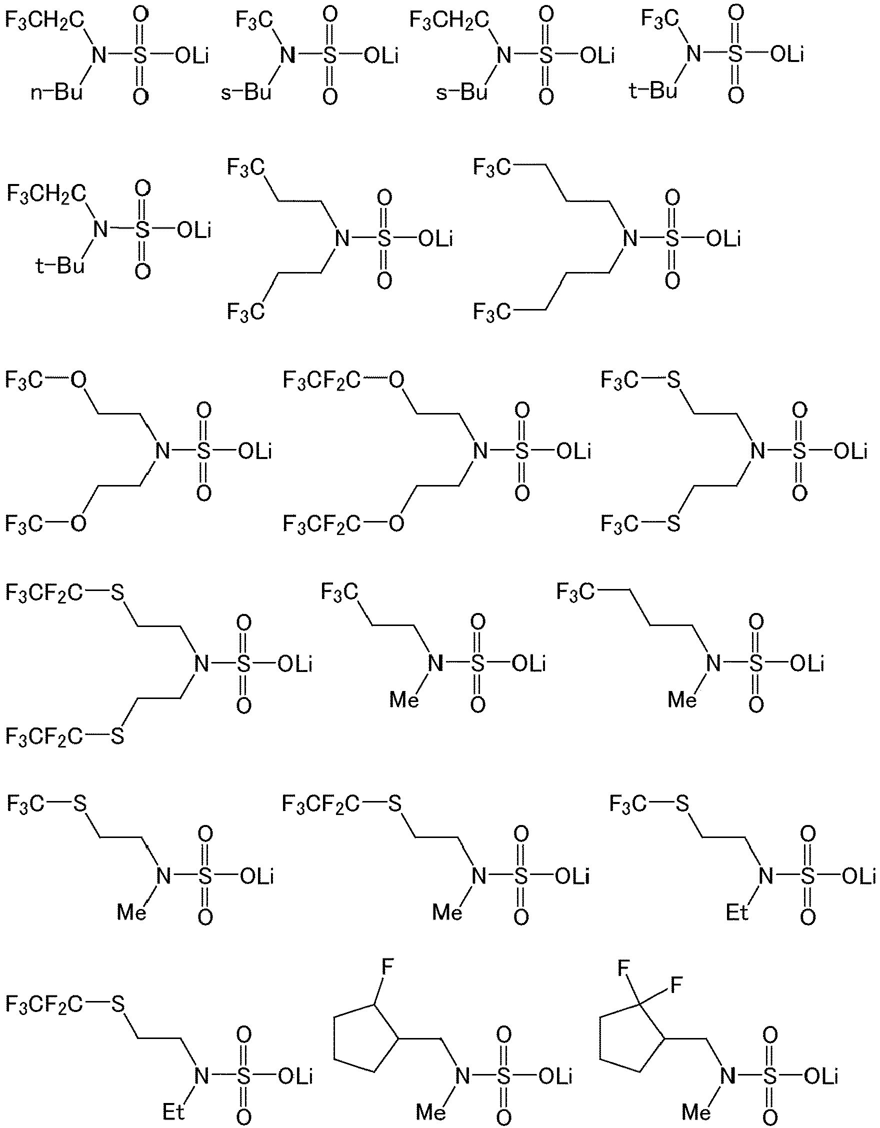

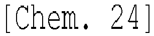

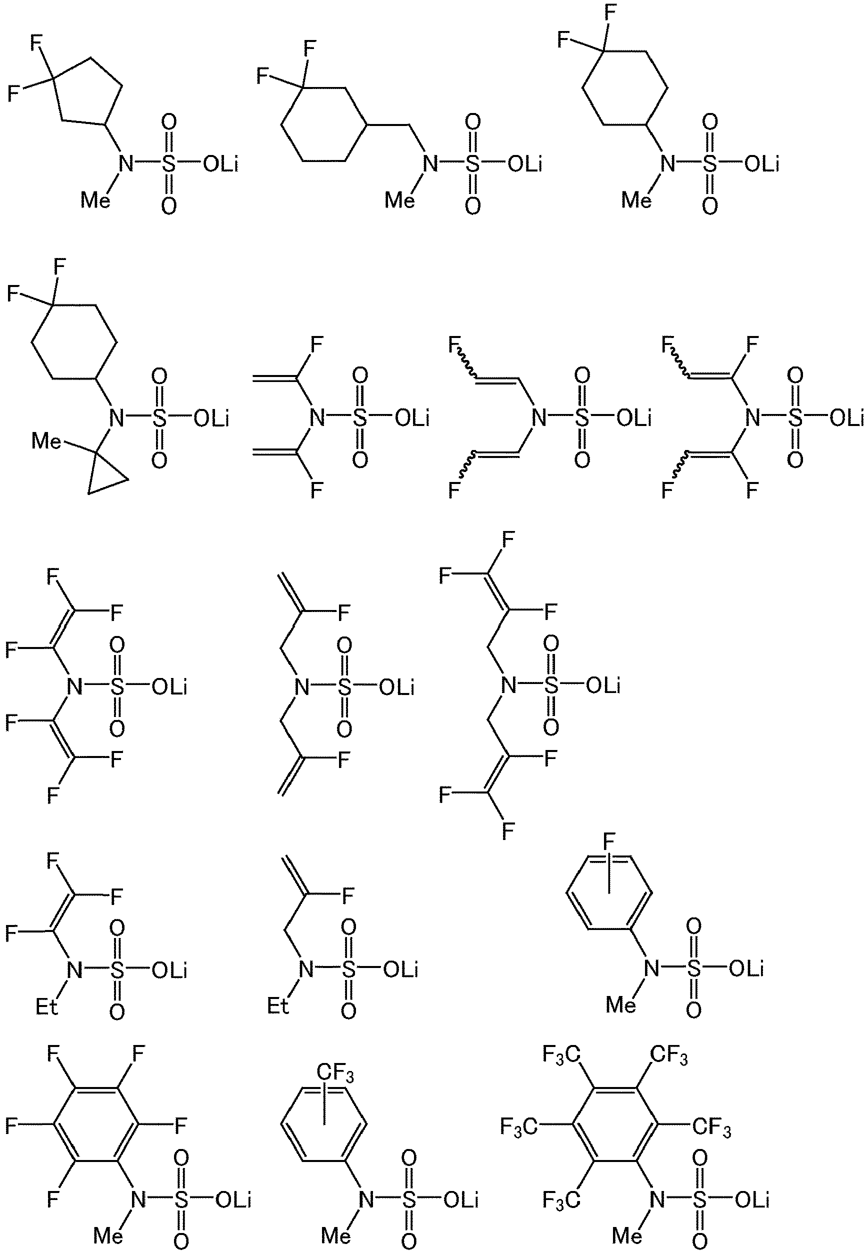

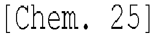

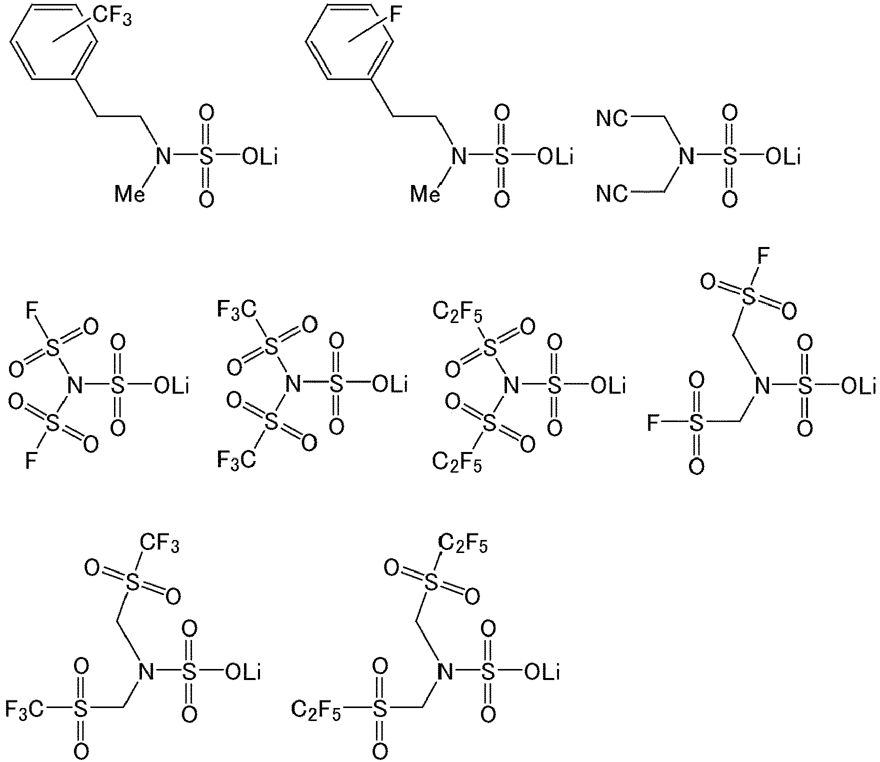

The amount of the compound (2) used in the step (1) is preferably at least 1.0 molar time, more preferably at least 1.1 molar times, still more preferably at least 1.5 molar times the amount of the compound (1). The upper limit is not limited. Still, the amount of the compound (2) is normally at most 3.0 molar times, preferably at most 2.5 molar times, more preferably at most 2.2 molar times the amount of the compound (1).

The reaction in the step (1) is preferably carried out in the presence of a base (excluding the compound (2)). Examples of the base include amines (excluding the compound (2)) and inorganic bases.

Examples of the amines include triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,8-diazabicyclo[5.4.0]-7-undecene (DBU), 1,5-diazabicyclo[4.3.0]-5-nonene, 1,4-diazabicyclo[2.2.2]octane (DABCO), 4-dimethylaminopyridine (DMAP), and proton sponge.

Examples of the inorganic bases include lithium hydroxide, potassium hydroxide, sodium hydroxide, calcium hydroxide, lithium carbonate, sodium carbonate, potassium carbonate, sodium hydrogen carbonate, potassium hydrogen carbonate, cesium carbonate, cesium hydrogen carbonate, lithium hydrogen carbonate, cesium fluoride, potassium fluoride, sodium fluoride, lithium chloride, and lithium bromide.

The base is preferably an amine. The amine is preferably triethylamine or pyridine.

The base may be solid or liquid at normal temperature. In the case where the base is solid, it can be dissolved in a solvent before use.

In the case where the base is used together, the total amount of the base and the compound (2) is preferably at least 2.0 molar times, more preferably at least 2.1 molar times, still more preferably at least 2.2 molar times the amount of the compound (1). The upper limit is not limited. Still, the total amount of the base and the compound (2) is normally at most 4.0 molar times, preferably at most 3.0 molar times, more preferably at most 2.6 molar times the amount of the compound (1). Here, the ratio between the base and the compound (2) (base:compound (2)) is within the range of preferably 0.01:0.99 to 0.60:0.40, more preferably 0.40:0.60 to 0.55:0.45, still more preferably 0.45:0.55 to 0.50:0.50.

The temperature during the step (1) is not limited as long as the reaction proceeds. For example, it is preferably 100°C or lower, more preferably 50°C or lower, still more preferably 30°C or lower. The temperature is preferably -50°C or higher, more preferably -30°C or higher, still more preferably -10°C or higher. Such a temperature is not likely to cause development of a side reaction and allows efficient progression of the reaction.

The reaction in the step (1) may be carried out in a solvent. The solvent is preferably a nonaqueous solvent. For example, preferred is a nonaqueous solvent having low reactivity with the compounds (1) and (2).

Also preferred is a nonaqueous solvent in which the compounds (1) and (2) are dissolved. For example, the nonaqueous solvent preferably has a solubility of the compound (1) at room temperature of 0.1% by mass or higher, more preferably 1% by mass or higher, still more preferably 5% by mass or higher.

The nonaqueous solvent preferably has a solubility of the compound (2) at room temperature of 0.1% by mass or higher, more preferably 1% by mass or higher, still more preferably 5% by mass or higher.

The solvent preferably has a boiling point at normal pressure of 300°C or lower, more preferably 200°C or lower, still more preferably 150°C or lower, because such a solvent is not likely to remain in the lithium sulfamate obtainable by the production method of the disclosure.

Specific examples of the solvent include: acyclic esters such as methyl acetate, ethyl acetate, ethyl methanesulfonate, and methyl ethanesulfonate; acyclic carbonates such as dimethyl carbonate, ethyl methyl carbonate, and diethyl carbonate; cyclic carbonates such as ethylene carbonate, propylene carbonate, and fluoroethylene carbonate; acyclic carboxylates such as methyl acetate, ethyl acetate, and methyl propionate; halogenated hydrocarbons such as dichloromethane, 1,2-dichloroethane, chloroform, and carbon tetrachloride; acyclic ethers such as diethyl ether, ethyl methyl ether, tert-butyl methyl ether, and dimethoxyethane; cyclic ethers such as tetrahydrofuran, 1,3-dioxane, and 1,4-dioxane; acyclic nitriles such as acetonitrile and propionitrile; and lactones, ketones, aldehydes, amides, and hydrocarbon solvents. From the standpoints of the miscibility with the compound (1) and the compound (2), the boiling point, and the availability thereof, preferred is dimethyl carbonate, ethyl methyl carbonate, diethyl carbonate, acetonitrile, dichloromethane, or chloroform, and more preferred is dimethyl carbonate or acetonitrile. These nonaqueous solvents may be used alone or in combination. Even a protic solvent is usable as long as it is a solvent such as a higher alcohol not reactive with the compounds (1), (2), and (3).

The ratio of the nonaqueous solvent to the compound (1) in the step (1) is not limited. Still, the weight of the nonaqueous solvent is preferably not more than 100 times, more preferably not more than 50 times, still more preferably not more than 25 times the weight of the compound (1). The weight of the nonaqueous solvent is preferably not less than 2 times, more preferably not less than 3 times, still more preferably not less than 5 times the weight of the compound (1). The weight ratio within the above range is not likely to cause precipitation of an unreacted compound (1) and further facilitate the production.

In the step (1), the compound (1) may be dropwise added to a solution of the compound (2) with stirring, or the compound (2) may be dropwise added to a solution of the compound (1). The compound (1) or (2) may be diluted before dropwise addition thereof. In the case where the compound (2) and a base are used together, the compound (2) and the base are preferably mixed in advance.

The production method of the first aspect of the disclosure preferably further includes a step (2) of reacting a compound (4) represented by the following formula (4): 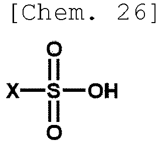 (wherein X is fluorine, chlorine, bromine, or iodine) with a lithium source to obtain the compound (1) represented by the following formula (1): 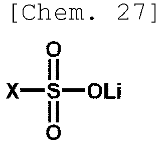 (wherein X is fluorine, chlorine, bromine, or iodine).

X in the formula (4) is fluorine, chlorine, bromine, or iodine. From the standpoints of the availability and reactivity of the compound (4) as a material, preferred is chlorine.

The lithium source in the step (2) is preferably lithium fluoride, lithium chloride, lithium bromide, lithium iodide, lithium hydride, n-butyllithium, sec-butyllithium, tert-butyllithium, lithium hydroxide, or metallic lithium, more preferably lithium fluoride, lithium chloride, lithium bromide, or lithium iodide, still more preferably lithium chloride.

The amount of the lithium source used in the step (2) is preferably at most 1.5 molar times, more preferably at most 1.2 molar times, still more preferably at most 1.0 molar time the amount of the compound (4). The lower limit is not limited. Still, the amount of the lithium source is normally at least 0.50 molar times, preferably at least 0.80 molar times, more preferably at least 0.90 molar times the amount of the compound (4).

The temperature during the step (2) is not limited as long as the reaction proceeds. For example, the temperature is preferably 150°C or lower, more preferably 120°C or lower, still more preferably 90°C or lower. The temperature is preferably -20°C or higher, more preferably 0°C or higher, still more preferably 20°C or higher. Such a temperature is not likely to cause development of a side reaction and allows efficient progression of the reaction.

Though the reaction in the step (2) may be carried out in the absence of a solvent, it may be carried out in a solvent. The solvent used is not limited as long as it is a non-aqueous solvent, and is more preferably an aprotic solvent. For example, preferred is an aprotic solvent having low reactivity with the compound (4).

Further, it is preferably an aprotic solvent capable of dissolving the compound (4) therein. For example, the solvent preferably has a solubility of the compound (4) at room temperature of 0.1% by mass or higher, more preferably 1% by mass or higher, still more preferably 5% by mass or higher.

The solvent preferably has a boiling point at normal pressure of 300°C or lower, more preferably 200°C or lower, still more preferably 150°C or lower, because such a solvent is not likely to remain in the lithium sulfamate obtainable by the production method of the disclosure.

Specific examples of the solvent include: acyclic esters such as methyl acetate, ethyl acetate, ethyl methanesulfonate, and methyl ethanesulfonate; acyclic carbonates such as dimethyl carbonate, ethyl methyl carbonate, and diethyl carbonate; cyclic carbonates such as ethylene carbonate, propylene carbonate, and fluoroethylene carbonate; acyclic carboxylates such as methyl acetate, ethyl acetate, and methyl propionate; halogenated hydrocarbons such as dichloromethane, 1,2-dichloroethane, chloroform, and carbon tetrachloride; acyclic ethers such as diethyl ether, ethyl methyl ether, tert-butyl methyl ether, and dimethoxyethane; cyclic ethers such as tetrahydrofuran, 1,3-dioxane, and 1,4-dioxane; acyclic nitriles such as acetonitrile and propionitrile; and lactones, ketones, aldehydes, amides, and hydrocarbon solvents. From the standpoints of the miscibility with the compound (4) and the lithium source, the boiling point, and the availability thereof, preferred is dimethyl carbonate, ethyl methyl carbonate, diethyl carbonate, acetonitrile, dichloromethane, or chloroform, and more preferred is dimethyl carbonate or acetonitrile. These nonaqueous solvents may be used alone or in combination. Even a protic solvent is usable as long as it is a solvent such as a higher alcohol not reactive with the compounds (4) and (1) .

The ratio of the nonaqueous solvent to the compound (4) in the step (2) is not limited. Still, the volume of the nonaqueous solvent is preferably not more than 100 times, more preferably not more than 50 times, still more preferably not more than 25 times the volume of the compound (4). The volume of the nonaqueous solvent is preferably not less than 1 time, more preferably not less than 3 times, still more preferably not less than 5 times the volume of the compound (4). The volume ratio within the above range, is not likely to cause precipitation of the compound (1) obtained and further facilitate the production thereof.

In the step (2), the lithium source may be dropwise added to a solution of the compound (4) with stirring, or the compound (4) may be dropwise added to the lithium source dissolved or suspended in a solvent. The compound (4) may be diluted upon dropwise addition. In the absence of a solvent, the lithium source may be added to the compound (4) or the compound (4) may be added to the lithium source. The lithium source may be used alone or in the form of a solution.

In the production method of the first aspect of the disclosure, the step (2) is carried out before the step (1). The production method may include, between the step (2) and the step (1), a step of recovering the compound (1) in the solvent obtained in the step (2), and may further include a purification step such as recrystallization.

In the case where the step (2) and the step (1) are continuously carried out in the same solvent, the recovery step or the purification step are not needed.

The production method of the first aspect of the disclosure may include, after the step (1), a step of recovering the compound (3) in the solvent obtained in the step (1), and may further include a purification step such as pH adjustment and recrystallization.

The production method of the second aspect of the disclosure includes (3) reacting a compound (4) represented by the following formula (4): 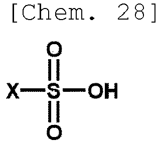 wherein X is fluorine, chlorine, bromine, or iodine, and a compound (5) represented by the following formula (5): 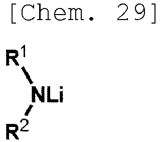 wherein R¹ and R² are defined as above, to obtain a compound (3) represented by the following formula (3): 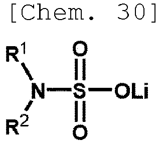 wherein R¹ and R² are defined as above.

The production method of the second aspect of the disclosure can provide a novel lithium sulfamate owing to the above configuration. Also, it can reduce heat of reaction, enabling simple and efficient production of a lithium sulfamate. The production method is also beneficial in that hardly separable by-products, such as lithium sulfate, are not likely to be generated.

X in the formula (4) is fluorine, chlorine, bromine, or iodine. From the standpoints of the availability and reactivity of the compound (4) as a material, preferred is chlorine.

Examples of the R¹ and R² include those exemplified for the compounds (2) and (3). From the standpoint of lowering the basic properties of the compound (5) to reduce the heat of reaction with the compound (4), preferred is a substituent including an electron-withdrawing substituent. The electron-withdrawing substituent is particularly preferably a fluorinated alkyl group, a fluorinated alkenyl group, a fluorinated alkynyl group, a sulfonyl group, a cyano group, or a cyanomethyl group.

Specific examples of the electron-withdrawing group include, but are not limited to, trifluoromethyl, 2,2,2-trifluoroethyl, pentafluoroethyl, 2,2,3,3,3-pentafluoropropyl, heptafluoropropyl, fluorosulfonyl, trifluoromethanesulfonyl, 2,2,2-trifluoroethanesulfonyl, pentafluoroethanesulfonyl, 2,2,3,3,3-pentafluoropropanesulfonyl, heptafluoropropanesulfonyl, cyano, and cyanomethyl groups.

Specific examples of the compound (5) include the following compounds.

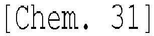

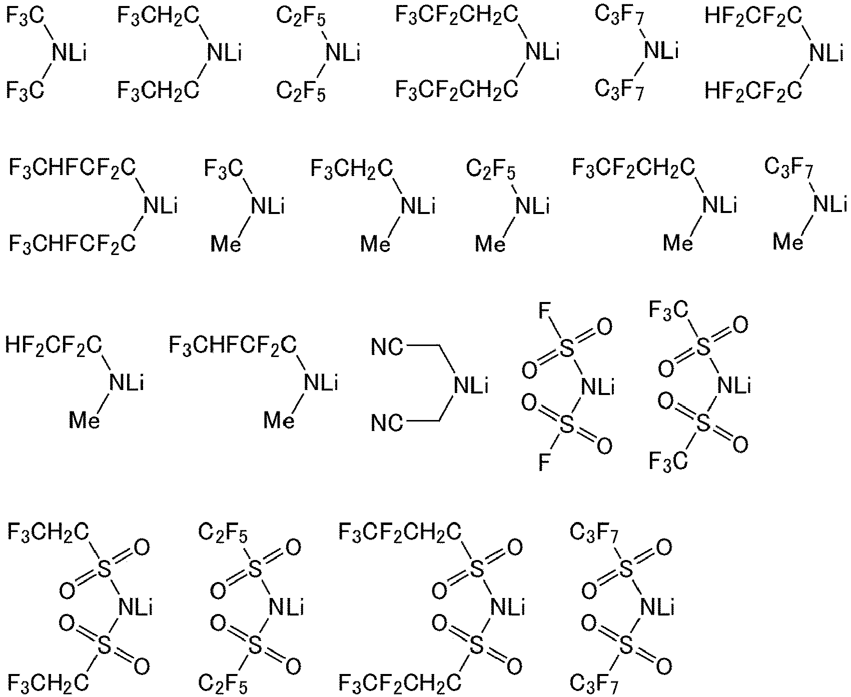

Specific examples of the compound (3) include the following compounds.

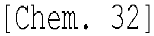

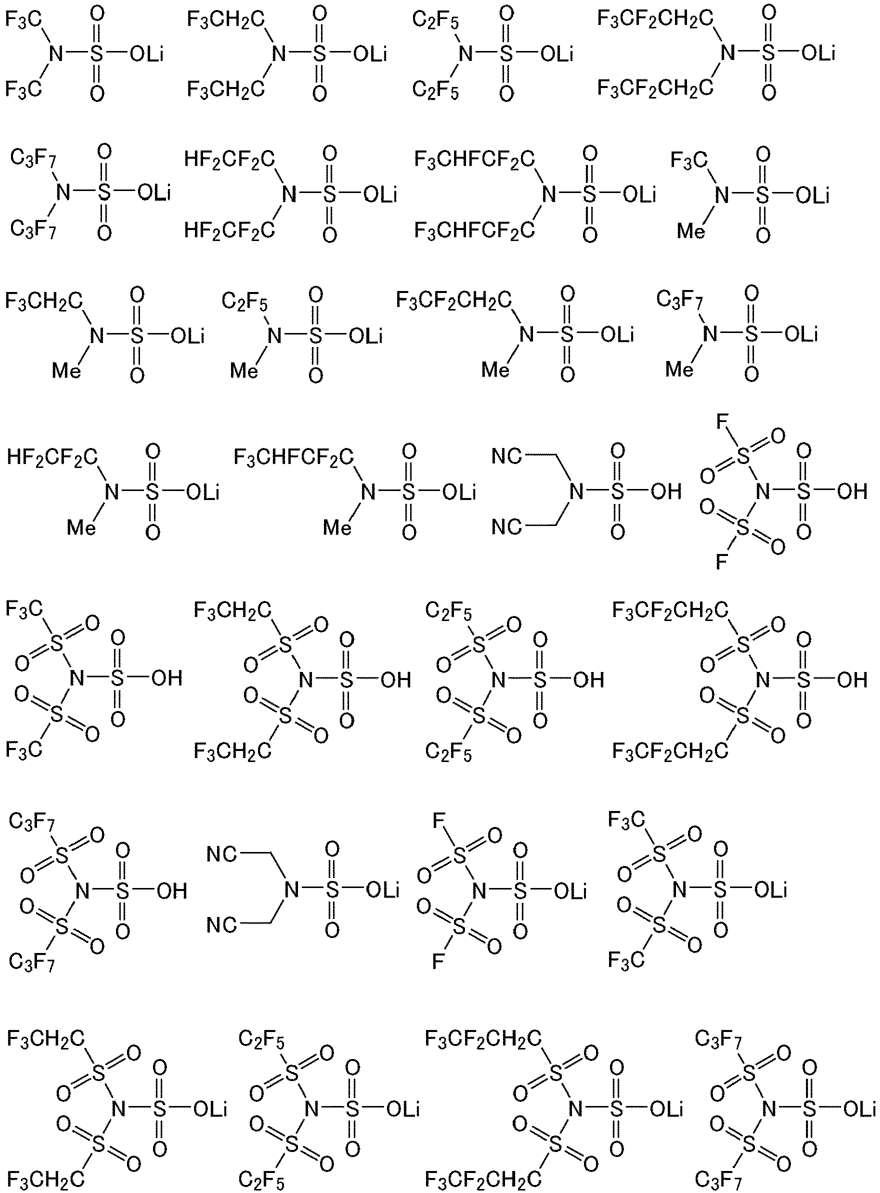

The amount of the compound (5) used in the step (3) is preferably at least 0.7 molar times, more preferably at least 0.8 molar times, still more preferably at least 0.9 molar times the amount of the compound (4). The upper limit is not limited. Still, the amount of the compound (5) is normally at most 2.0 molar times, more preferably at most 1.5 molar times, still more preferably at most 1.1 molar times the amount of the compound (4).

The temperature during the step (3) is not limited as long as the reaction proceeds. For example, it is preferably 200°C or lower, more preferably 170°C or lower, still more preferably 150°C or lower. The temperature is preferably 0°C or higher, more preferably 20°C or higher, still more preferably 50°C or higher. Such a temperature allows efficient progression of the reaction.

The reaction in the step (3) may be carried out in a solvent. The solvent is preferably a nonaqueous solvent. For example, preferred is a nonaqueous solvent having low reactivity with the compounds (4), (5), and (3).

Also preferred is a nonaqueous solvent in which the compounds (4) and (5) are dissolved. For example, the nonaqueous solvent preferably has a solubility of the compound (4) at room temperature of 0.1% by mass or higher, more preferably 1% by mass or higher, still more preferably 5% by mass or higher.

The nonaqueous solvent preferably has a solubility of the compound (5) at room temperature of 0.1% by mass or higher, more preferably 1% by mass or higher, still more preferably 5% by mass or higher.

The solvent preferably has a boiling point at normal pressure of 300°C or lower, more preferably 200°C or lower, still more preferably 150°C or lower, because such a solvent is not likely to remain in the lithium sulfamate obtainable by the production method of the disclosure.

Specific examples of the solvent include: acyclic esters such as methyl acetate, ethyl acetate, ethyl methanesulfonate, and methyl ethanesulfonate; acyclic carbonates such as dimethyl carbonate, ethyl methyl carbonate, and diethyl carbonate; cyclic carbonates such as ethylene carbonate propylene carbonate, and fluoroethylene carbonate; acyclic carboxylates such as methyl acetate, ethyl acetate, and methyl propionate; halogenated hydrocarbons such as dichloromethane, 1,2-dichloroethane, chloroform, and carbon tetrachloride; acyclic ethers such as diethyl ether, ethyl methyl ether, tert-butyl methyl ether, and dimethoxyethane; cyclic ethers such as tetrahydrofuran, 1,3-dioxane, and 1,4-dioxane; acyclic nitriles such as acetonitrile and propionitrile; and lactones, ketones, aldehydes, amides, and hydrocarbon solvents. From the standpoints of the miscibility with the compounds (4) and (5), the boiling point, and availability thereof, preferred is dimethyl carbonate, ethyl methyl carbonate, diethyl carbonate, acetonitrile, or diethyl ether, and more preferred is dimethyl carbonate, acetonitrile, or diethyl ether. These nonaqueous solvents may be used alone or in combination. Even a protic solvent is usable as long as it is a solvent such as a higher alcohol not reactive with the compounds (4), (5), and (3).

The ratio of the nonaqueous solvent to the compound (4) in the step (3) is not limited. Still, the volume of the nonaqueous solvent is preferably not more than 100 times, more preferably not more than 50 times, still more preferably not more than 25 times the volume of the compound (4). The volume of the nonaqueous solvent is preferably not less than 1 time, more preferably not less than 3 times, still more preferably not less than 5 times the volume of the compound (4). The volume ratio within the above range is not likely to develop side reactions, and further facilitate the production of the compound (3).

In the step (3), the compound (5) may be dropwise added to a solution of the compound (4) with stirring, or the compound (4) may be dropwise added to a solution of the compound (5). The compound (4) or (5) may be diluted before dropwise addition thereof.

The production method of the second aspect of the disclosure may include, after the step (3), a step of recovering the compound (3) in the solvent obtained in the step (3), and may further include a purification step such as pH adjustment and recrystallization.

The disclosure provides a lithium sulfamate that is a novel compound.

The novel compound of the disclosure is represented by the following formula (3a): 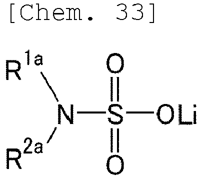 wherein R^{1a} and R^{2a} are each individually a substituent that is:
- H;
   a group represented by the formula: -Oₚ-(SiR³₂O)ₙ-SiR⁴₃ (R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom; n is an integer of 0 or greater; and p is 0 or 1);
   a C1-C7 alkyl group;
   a C2-C7 alkenyl group;
   a C2-C7 alkynyl group;
- SO₂X¹ (X¹ is -H, -F, or an alkyl group in which at least one hydrogen atom is optionally replaced with a fluorine atom);
- SO₃X² (X² is -H, -F, or an alkyl group in which at least one hydrogen atom is optionally replaced with a fluorine atom); or
   a C2-C7 hydrocarbon group that forms a cyclic structure by bonding of R^{1a} and R^{2a}, the cyclic structure optionally containing a multiple bond,
   the substituent optionally containing at least one bi- to hexavalent heteroatom in the structure and being a substituent in which at least one hydrogen atom is optionally replaced with a fluorine atom or a C0-C7 functional group, and
   when R^{1a} and R^{2a} are both alkyl groups, at least one alkyl group contains at least one bi- to hexavalent heteroatom in the structure or is an alkyl group in which at least one hydrogen atom is optionally replaced with a fluorine atom or a C0-C7 functional group.

In the formula (3a), examples of R^{1a} and R^{2a} include those corresponding to R^{1a} and R^{2a} among the groups exemplified as R¹ and R² in the above production methods.

In the formula (3a), preferably, R^{1a} and R^{2a} are each individually a substituent that is a C1-C7 alkyl group in which at least one hydrogen atom is replaced with a fluorine atom, a C2-C7 alkenyl group in which at least one hydrogen atom is optionally replaced with a fluorine atom, a C2-C7 alkynyl group in which at least one hydrogen atom is optionally replaced with a fluorine atom, -SO₂X (X is -H, -F, or an alkyl group in which at least one hydrogen atom is optionally replaced with a fluorine atom), a cyanomethyl group, or a C2-C7 hydrocarbon group that forms a cyclic structure by bonding of R^{1a} and R^{2a}, the cyclic structure optionally containing a multiple bond and at least one hydrogen atom therein being optionally replaced with a fluorine atom. The substituent optionally contains at least one oxygen, sulfur, or nitrogen atom in the structure.

In the formula (3a), preferably, R^{1a} and R^{2a} are each individually a 4-tetrahydropyranyl group, an allyl group, a propargyl group, a butane-1,4-diyl group, a pentane-1,5-diyl group, a 3-oxapentane-1,5-diyl group, a trifluoromethyl group, a 2,2,2-trifluoroethyl group, a 1,1,2,2-tetrafluoroethyl group, a pentafluoroethyl group, a 2,2,3,3,3-pentafluoropropyl group, a 1,1,2,3,3,3-hexafluoropropyl group, a heptafluoropropyl group, a 2-fluoroallyl group, a fluorophenyl group, a cyanomethyl group, a fluorosulfonyl group, a trifluoromethanesulfonyl group, a pentafluoroethanesulfonyl group, a 2,2,2-trifluoroethanesulfonyl group, or a 2,2,3,3,3-pentafluoropropanesulfonyl group.

From the standpoint of the solubility of a lithium sulfamate, R^{1a} and R^{2a} are each more preferably an allyl group, a propargyl group, a butane-1,4-diyl group, a pentane-1,5-diyl group, a 3-oxapentane-1,5-diyl group, a trifluoromethyl group, a 2,2,2-trifluoroethyl group, a 1,1,2,2-tetrafluoroethyl group, a 2,2,3,3,3-pentafluoropropyl group, a 1,1,2,3,3,3-hexafluoropropyl group, a 2-fluoroallyl group, a cyanomethyl group, a fluorosulfonyl group, a trifluoromethanesulfonyl group, or a pentafluoroethanesulfonyl group.

Specific examples of the compound include the following compounds.

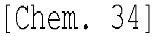

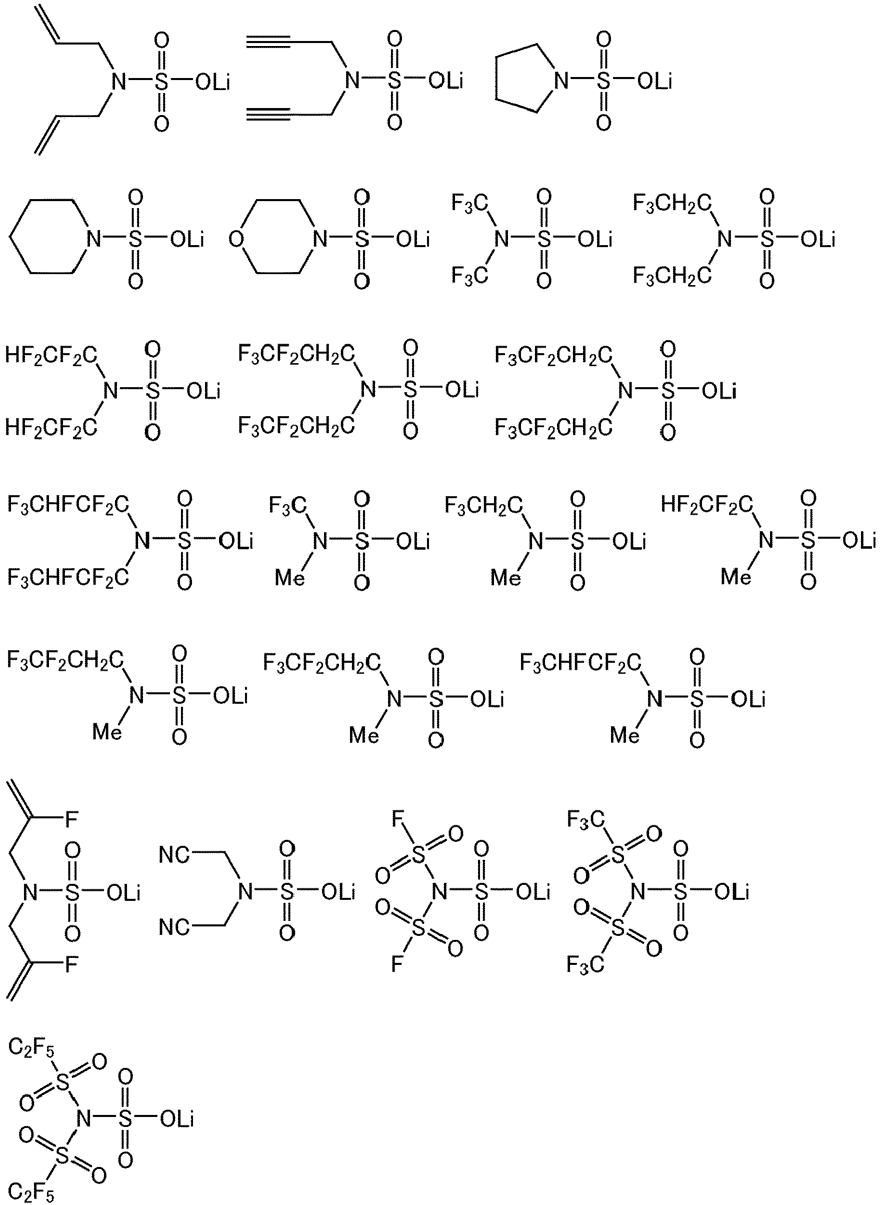

The novel lithium sulfamate of the disclosure can be used as a functional compound such as an intermediate of medicine, a surfactant, or a food additive.

### EXAMPLES

The lithium sulfamate of the disclosure is described with reference to, but not limited to, examples.

The structures of the compounds obtained in the examples were specified by ¹H- or ¹⁹F-NMR.

### Example 1

### <Synthesis of lithium chlorosulfonate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, cooled, and filtered to obtain target lithium chlorosulfonate (8.0 g, 65 mmol, yield of 92%).

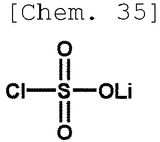

### <Synthesis of lithium diethyl sulfamate>

A reaction vessel was charged with the lithium chlorosulfonate (2.0 g, 16 mmol) and dimethyl carbonate (20 mL). A mixed solution containing diethylamine (1.2 g, 16 mmol) and triethylamine (1.7 g, 16 mmol) was dropwise added thereto. The obtained solution was stirred at room temperature for 30 minutes, and then filtered. The residue was washed with dichloromethane to obtain target lithium diethyl sulfamate (0.9 g, 6 mmol, yield of 35%).

¹H-NMR (deuterated methanol, δ ppm): 1.10 to 1.13 (t), 3.07 to 3.13 (q)

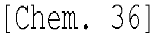

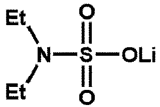

### Example 2

### <Synthesis of lithium diethyl sulfamate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, and cooled to room temperature. A mixed solution containing diethylamine (6.2 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain target coarse lithium diethyl sulfamate (9.1 g). The obtained coarse lithium diethyl sulfamate (2.0 g) was reprecipitated using methanol (7 mL) and dimethyl carbonate (7 mL) to obtain target lithium diethyl sulfamate (0.9 g, 6 mmol, total yield of 38%). ¹H-NMR (deuterated methanol, δ ppm): 1.10 to 1.13 (t), 3.07 to 3.13 (q)

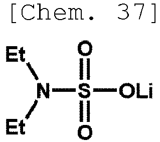

### Example 3

### <Synthesis of lithium diisopropyl sulfamate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, and cooled to room temperature. A mixed solution containing diisopropylamine (8.6 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain target lithium diisopropyl sulfamate (10.6 g, 57 mmol, yield of 80%). ¹H-NMR (deuterated methanol, δ ppm): 1.21 to 1.23 (d), 3.63 to 3.66 (sep)

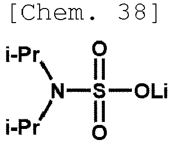

### Example 4

### <Synthesis of lithium diallyl sulfamate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, and cooled to room temperature. A mixed solution containing diallylamine (8.3 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain target coarse lithium diallyl sulfamate (8.7 g). The obtained coarse lithium diallyl sulfamate (8.7 g) was reprecipitated using methanol (14 mL) and dimethyl carbonate (14 mL) to obtain target lithium diallyl sulfamate (3.5 g, 19 mmol, total yield of 27%). ¹H-NMR (deuterated methanol, δ ppm): 3.65 to 3.67 (m), 5.07 to 5.14 (m), 5.86 to 5.94 (m)

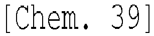

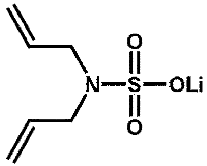

### Example 5

### <Synthesis of lithium pentane-1,5-diyl sulfamate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, and cooled to room temperature. A mixed solution containing piperidine (7.2 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain target coarse lithium pentane-1,5-diyl sulfamate (10.3 g). The obtained coarse lithium pentane-1,5-diyl sulfamate (10.3 g) was reprecipitated using methanol (16 mL) and dimethyl carbonate (16 mL) to obtain target lithium pentane-1,5-diyl sulfamate (4.7 g, 27 mmol, total yield of 39%) .

¹H-NMR (deuterated methanol, δ ppm): 1.43 to 1.48 (m), 1.57 to 1.62 (m), 2.99 to 3.02 (m)

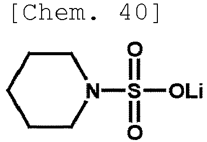

### Example 6

### <Synthesis of lithium 3-oxapentane-1,5-diyl sulfamate>

A reaction vessel was charged with lithium chloride (3.0 g, 71 mmol) and dimethyl carbonate (60 mL).

Chlorosulfonic acid (9.1 g, 78 mmol) was dropwise added thereto. The obtained solution was stirred at 80°C for one hour, and cooled to room temperature. A mixed solution containing morpholine (7.4 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain target lithium 3-oxapentane-1,5-diyl sulfamate (12.5 g). The obtained coarse lithium 3-oxapentane-1,5-diyl sulfamate (12.5 g) was reprecipitated using methanol (140 mL) and dimethyl carbonate (140 mL) to obtain target lithium 3-oxapentane-1,5-diyl sulfamate (3.1 g, 18 mmol, total yield of 25%).

¹H-NMR (deuterated methanol, δ ppm): 3.00 to 3.02 (m), 3.67 to 3.72 (m)

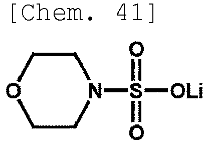

### Example 7

### <Synthesis of lithium butane-1,4-diyl sulfamate>

A reaction vessel was charged with lithium chloride (3.0 g, 71 mmol) and dimethyl carbonate (60 mL). Chlorosulfonic acid (9.1 g, 78 mmol) was dropwise added thereto. The obtained solution was stirred at 80°C for one hour, and cooled to room temperature. A mixed solution containing pyrrolidine (6.0 g, 85 mmol) and triethylamine (8.6 g, 85 mmol) was dropwise added to the solution in an ice water bath (temperature rise of reaction solution: 5°C to 10°C). The solution was stirred at room temperature for one hour, and then filtered. The residue was washed with dichloromethane to obtain coarse lithium butane-1,4-diyl sulfamate (9.7 g). The obtained coarse lithium butane-1,4-diyl sulfamate (9.7 g) was reprecipitated using methanol (100 mL) and dimethyl carbonate (100 mL) to obtain target lithium butane-1,4-diyl sulfamate (3.7 g, 24 mmol, total yield of 33%).

¹H-NMR (deuterated methanol, δ ppm): 1.78 to 1.86 (m), 3.09 to 3.18 (m)

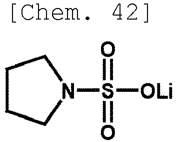

### Example 8

### <Synthesis of lithium methyl 2,2,2-trifluoroethyl sulfamate>

A reaction vessel was charged with lithium chloride (1.0 g, 24 mmol) and dimethyl carbonate (35 mL). Chlorosulfonic acid (3.0 g, 26 mmol) was dropwise added thereto. The obtained solution was stirred at 80°C for one hour, and cooled to room temperature. Methyl 2,2,2-trifluoroethylamine (6.4 g, 57 mmol) was dropwise added to the solution in an ice water bath (temperature rise of reaction solution: 0°C to 5°C). The solution was stirred at room temperature for one hour, and triethylamine (6.0 g) and dichloromethane (50 mL) were added, followed by further stirring for one day. The obtained reaction mixture was filtered, and the residue was washed with dichloromethane to obtain target lithium methyl 2,2,2-trifluoroethyl sulfamate (2.3 g, 12 mmol, total yield of 49%).

¹H-NMR (deuterated methanol, δ ppm): 2.79 (s), 3.53 to 3.60 (q)

¹⁹F-NMR (deuterated methanol, δ ppm): -72.35 to -72.30 (m)

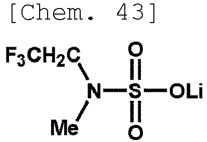

### Example 9

### <Synthesis of lithium bis(2,2,2-trifluoroethyl) sulfamate>

A reaction vessel was charged with lithium chloride (1.0 g, 24 mmol) and dimethyl carbonate (35 mL). Chlorosulfonic acid (3.0 g, 26 mmol) was dropwise added thereto. The obtained solution was stirred at 80°C for one hour, and cooled to room temperature. Bis(2,2,2-trifluoroethyl)amine (10.3 g, 57 mmol) was dropwise added to the solution in an ice water bath (temperature rise of reaction solution: 0°C to 5°C). The solution was stirred at room temperature for one hour, and triethylamine (6.0 g) and dichloromethane (50 mL) were added, followed by further stirring for one day. The obtained reaction mixture was filtered, and the residue was washed with dichloromethane to obtain target lithium bis(2,2,2-trifluoroethyl) sulfamate (3.4 g, 13 mmol, total yield of 54%).

¹H-NMR (deuterated methanol, δ ppm): 3.80 to 3.83 (q) ¹⁹F-NMR (deuterated methanol, δ ppm): -71.34 to -71.23 (m)

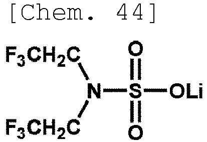

### Example 10

### <Synthesis of lithium bis(2,2,3,3,3-pentafluoropropyl) sulfamate>

A reaction vessel was charged with lithium chloride (1.0 g, 24 mmol) and dimethyl carbonate (35 mL). Chlorosulfonic acid (3.0 g, 26 mmol) was dropwise added thereto. The obtained solution was stirred at 80°C for one hour, and cooled to room temperature. Bis(2,2,3,3,3-pentafluoropropyl)amine (13.3 g, 57 mmol) was dropwise added to the solution in an ice water bath (temperature rise of reaction solution: 0°C to 5°C). The solution was stirred at room temperature for one hour, and triethylamine (6.0 g) and dichloromethane (50 mL) were added, followed by further stirring for one day. The obtained reaction mixture was filtered, and the residue was washed with dichloromethane to obtain target lithium bis(2,2,3,3,3-pentafluoropropyl) sulfamate (6.3 g, 17.2 mmol, yield of 72%) .

¹H-NMR (deuterated methanol, δ ppm): 3.87 to 3.95 (t) ¹⁹F-NMR (deuterated methanol, δ ppm): -121.10 to -120.96 (m), -86.24 to -86.21 (m)

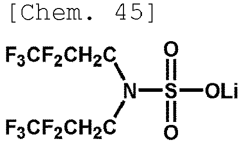

### Example 11

### <Synthesis of lithium bis(cyanomethyl) sulfamate>

A reaction vessel was charged with lithium chloride (1.0 g, 24 mmol) and dimethyl carbonate (35 mL). Chlorosulfonic acid (3.0 g, 26 mmol) was dropwise added thereto. The obtained solution was stirred at 80°C for one hour, and cooled to room temperature. A mixed solution containing bis(cyanomethyl)amine (5.4 g, 57 mmol) dissolved in acetonitrile (30 mL) was dropwise added to the solution in an ice water bath (temperature rise of reaction solution: 5°C to 10°C). The solution was stirred at room temperature for one hour, and triethylamine (6.0 g) and dichloromethane (50 mL) were added, followed by further stirring for one day. The obtained reaction mixture was filtered, and the residue was washed with dichloromethane to obtain target lithium bis(cyanomethyl) sulfamate (3.9 g, 22 mmol, total yield of 91%).

¹H-NMR (deuterated methanol, δ ppm): 4.18 (s)

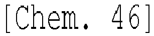

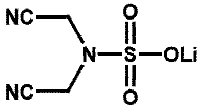

### Example 12

### <Synthesis of lithium bis(fluorosulfonyl) sulfamate>

A reaction vessel was charged with lithium bis(fluorosulfonyl)imide (3.5 g, 19 mmol) and diethyl ether (30 mL). Chlorosulfonic acid (2.2 g, 19 mmol) was dropwise added thereto (no heat generated). The obtained solution was stirred at room temperature for one day, and the solvent was distilled off from the solution to obtain a mixture (4.5 g) containing target lithium bis(fluorosulfonyl) sulfamate.

¹⁹F-NMR (deuterated methanol, δ ppm): 55.70 (s)

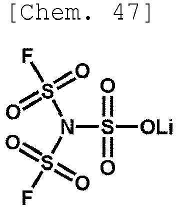

### Example 13

### <Synthesis of lithium bis(trifluoromethane sulfonyl) sulfamate>

A reaction vessel was charged with lithium bis(trifluoromethane sulfonyl)imide (3.9 g, 14 mmol) and diethyl ether (30 mL). Chlorosulfonic acid (1.6 g, 14 mmol) was dropwise added thereto (no heat generated). The obtained solution was stirred at room temperature for one day, and the solvent was distilled off from the solution to obtain a mixture (4.5 g) containing target lithium bis(trifluoromethane sulfonyl) sulfamate.

¹⁹F-NMR (deuterated methanol, δ ppm): -74.08 (s)

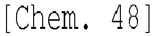

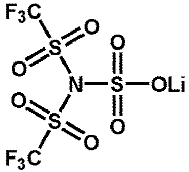

### Example 14

### <Synthesis of lithium bis(2,2,2-trifluoroethyl) sulfamate>

A reaction vessel was charged with preliminarily prepared lithium bis(2,2,2-trifluoroethyl)imide (2.1 g, 11 mmol) and tetrahydrofuran (60 mL), and cooled to -15°C. Chlorosulfonic acid (1.3 g, 11 mmol) was dropwise added thereto (temperature rise of reaction solution: 0°C to 5°C). The obtained solution was stirred at room temperature for one day, and the solvent was distilled off from the solution to obtain target lithium bis(2,2,2-trifluoroethyl) sulfamate (2.2 g, 8.1 mmol, yield of 74%).

¹H-NMR (deuterated methanol, δ ppm): 3.80 to 3.83 (q) ¹⁹F-NMR (deuterated methanol, δ ppm): -71.34 to -71.23 (m)

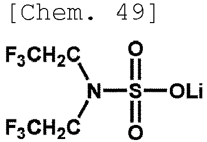

### Comparative Example 1

### <Synthesis of lithium diethyl sulfamate>

A reaction vessel was charged with diethylamine (6.0 g, 82 mmol) and dichloromethane (30 mL). Chlorosulfonic acid (10.5 g, 90 mmol) was dropwise added thereto while the reaction solution was maintained to have a temperature of - 30°C to -10°C in a dry ice/acetone bath (temperature rise of reaction solution by addition of a single droplet of the chlorosulfonic acid: 10°C to 15°C). The obtained solution was stirred for five hours while it was naturally warmed to room temperature. Then, the solvent was distilled off from the solution, and diethyl ether (120 mL) was added thereto, followed by stirring for one day. The mixture was washed, and precipitated solids were collected by filtration to obtain a mixture (10.8 g) containing diethylsulfamic acid. A 1-g portion of the obtained mixture was put into a reaction vessel, and a 0.8 M lithium hydroxide aqueous solution (8 mL) was added thereto, followed by stirring for three hours. The solvent was distilled off from the solution to obtain a white solid. As a result of ¹H-NMR measurement of the obtained solid, generation of target lithium diethyl sulfamate (NMR yield of about 10%) was confirmed.

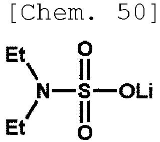

### Comparative Example 2

### <Synthesis of lithium dimethyl sulfamate>

A reaction vessel was charged with ice water (40 mL), and dimethylsulfamoyl chloride (5.0 g, 35 mmol) was dropwise added thereto. The mixture was stirred for 30 minutes and the solvent was distilled off. Thus, a mixture containing dimethylsulfamic acid was obtained. A 1.0 M lithium hydroxide aqueous solution (35 mL) was added thereto, followed by stirring for one hour. The solvent was distilled off from the solution to obtain a white solid. As a result of ¹H-NMR measurement and ion chromatography of the obtained solid, generation of a mixture containing target lithium dimethyl sulfamate and lithium sulfate (2.4 g, yield of 53% or lower) was confirmed.

¹H-NMR (deuterated methanol, δ ppm): 2.61 (s)

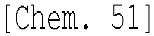

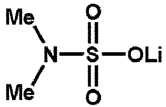

### EMBODIMENTS

Embodiment 1. A method for producing a lithium sulfamate, comprising
   (1) reacting a compound (1) represented by the following formula (1): 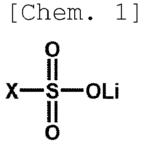 wherein X is fluorine, chlorine, bromine, or iodine, with a compound (2) represented by the following formula (2) : 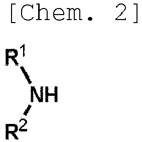 wherein R¹ and R² are each individually a substituent that is:
      - H;
         a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1;
         a C1-C7 alkyl group;
         a C2-C7 alkenyl group;
         a C2-C7 alkynyl group;
         a C6-C15 aryl group;
      - SO₂X¹ wherein X¹ is -H, -F, or an alkyl group in which at least one hydrogen atom is optionally replaced with a fluorine atom;
      - SO₃X² wherein X² is -H, -F, or an alkyl group in which at least one hydrogen atom is optionally replaced with a fluorine atom; or
         a C2-C7 hydrocarbon group that forms a cyclic structure by bonding of R¹ and R², the cyclic structure optionally containing a multiple bond,
         the substituent optionally containing at least one bi- to hexavalent heteroatom in the structure and being a substituent in which at least one hydrogen atom is optionally replaced with a fluorine atom or a C0-C7 functional group, to obtain a compound (3) represented by the following formula (3): 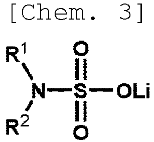 wherein R¹ and R² are defined as above.
Embodiment 2. The production method according to embodiment 1, further comprising
   (2) reacting a compound (4) represented by the following formula (4): 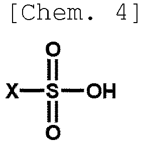 wherein X is fluorine, chlorine, bromine, or iodine, with a lithium source to obtain the compound (1) represented by the following formula (1): 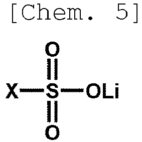 wherein X is fluorine, chlorine, bromine, or iodine.
Embodiment 3. The production method according to embodiment 1 or 2,
   wherein the lithium source is lithium fluoride, lithium chloride, lithium bromide, lithium iodide, lithium hydride, lithium hydroxide, or metallic lithium.
Embodiment 4. The production method according to embodiment 1, 2, or 3,
   wherein the lithium source is lithium fluoride, lithium chloride, lithium bromide, or lithium iodide.
Embodiment 5. A method for producing a lithium sulfamate, comprising
   (3) reacting a compound (4) represented by the following formula (4): 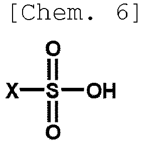 wherein X is fluorine, chlorine, bromine, or iodine, with a compound (5) represented by the following formula (5) : 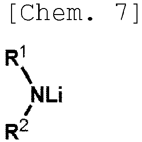 wherein R¹ and R² are each individually a substituent that is:
   - H;
      a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom, or an aryl group in which at least one hydrogen atom is optionally replaced with a fluorine atom; n is an integer of 0 or greater; and p is 0 or 1;
      a C1-C7 alkyl group;
      a C2-C7 alkenyl group;
      a C2-C7 alkynyl group;
      a C6-C15 aryl group;
   - SO₂X¹ wherein X¹ is -H, -F, or an alkyl group in which at least one hydrogen atom is optionally replaced with a fluorine atom;
   - SO₃X² wherein X² is -H, -F, or an alkyl group in which at least one hydrogen atom is optionally replaced with a fluorine atom; or
      a C2-C7 hydrocarbon group that forms a cyclic structure by bonding of R¹ and R², the cyclic structure optionally containing a multiple bond,
      the substituent optionally containing at least one bi- to hexavalent heteroatom in the structure and being a substituent in which at least one hydrogen atom is optionally replaced with a fluorine atom or a C0-C7 functional group, to obtain a compound (3) represented by the following formula (3): 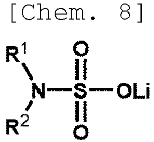 wherein R¹ and R² are defined as above.
Embodiment 6. The production method according to embodiment 5,
   wherein at least one selected from R¹ and R² in the compound (5) represented by the formula (5) is a trifluoromethyl group, a 2,2,2-trifluoroethyl group, a pentafluoroethyl group, a 2,2,3,3,3-pentafluoropropyl group, a heptafluoropropyl group, a fluorosulfonyl group, a trifluoromethanesulfonyl group, a 2,2,2-trifluoroethanesulfonyl group, a pentafluoroethanesulfonyl group, a 2,2,3,3,3-pentafluoropropanesulfonyl group, a heptafluoropropanesulfonyl group, or a cyanomethyl group.
Embodiment 7. A compound represented by the following formula (3a): 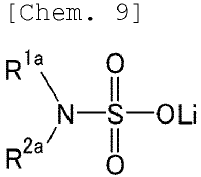 wherein R^{1a} and R^{2a} are each individually a substituent that is:
   - H;
      a group represented by the formula: -Oₚ-(SiR³₂O)ₙ-SiR⁴₃ wherein R³ and R⁴ are each individually an alkyl group in which at least one hydrogen atom is optionally replaced with a fluorine atom, an alkenyl group in which at least one hydrogen atom is optionally replaced with a fluorine atom, an alkynyl group in which at least one hydrogen atom is optionally replaced with a fluorine atom; n is an integer of 0 or greater; and p is 0 or 1;
      a C1-C7 alkyl group;
      a C2-C7 alkenyl group;
      a C2-C7 alkynyl group;
   - SO₂X¹ wherein X¹ is -H, -F, or an alkyl group in which at least one hydrogen atom is optionally replaced with a fluorine atom;
   - SO₃X² wherein X² is -H, -F, or an alkyl group in which at least one hydrogen atom is optionally replaced with a fluorine atom; or
      a C2-C7 hydrocarbon group that forms a cyclic structure by bonding of R^{1a} and R^{2a}, the cyclic structure optionally containing a multiple bond,
      the substituent optionally containing at least one bi- to hexavalent heteroatom in the structure and being a substituent in which at least one hydrogen atom is optionally replaced with a fluorine atom or a C0-C7 functional group, and
      when R^{1a} and R^{2a} are both alkyl groups, at least one alkyl group contains at least one bi- to hexavalent heteroatom in the structure or is an alkyl group in which at least one hydrogen atom is optionally replaced with a fluorine atom or a C0-C7 functional group.
Embodiment 8. The compound according to Embodiment 7,
   wherein in the formula (3a), R^{1a} and R^{2a} are each individually a substituent that is:
   a C1-C7 alkyl group in which at least one hydrogen atom is replaced with a fluorine atom;
   a C2-C7 alkenyl group in which at least one hydrogen atom is optionally replaced with a fluorine atom;
   a C2-C7 alkynyl group in which at least one hydrogen atom is optionally replaced with a fluorine atom;
      - SO₂X wherein X is -H, -F, or an alkyl group in which at least one hydrogen atom is optionally replaced with a fluorine atom;
   a cyanomethyl group; or
   a C2-C7 hydrocarbon group that forms a cyclic structure by bonding of R^{1a} and R^{2a}, the cyclic structure optionally containing a multiple bond and at least one hydrogen atom therein being optionally replaced with a fluorine atom,
   the substituent optionally containing at least one selected from oxygen, sulfur, and nitrogen atoms in the structure.

## Claims

1. A compound of formula (3a): 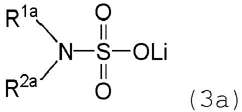 wherein R^{1a} and R^{2a} each independently are C₁₋₇-fluoroalkyl, C₂₋₇-alkenyl or C₂₋₇-alkynyl, each optionally fluorinated; -SO₂X wherein X is H, F or optionally fluorinated alkyl; or cyanomethyl;
and R^{1a} and R^{2a} each independently may contain at least one of O, S and N atoms in the structure.
